(19) Europäisches Patentamt
European Patent Office
Office européen des brevets

(11) Numéro de publication: **0 300 898 B1**

(12) **FASCICULE DE BREVET EUROPEEN**

(45) Date de publication de fascicule du brevet: **10.03.93**

(51) Int. Cl.5: **C07D 277/34**, C07D 277/10, C07D 277/36, C07D 263/38, C07D 263/46, C07D 263/32, C07D 417/12, C07D 413/12, A01N 53/00, A01N 43/78

(21) Numéro de dépôt: **88401875.5**

(22) Date de dépôt: **20.07.88**

Le dossier contient des informations techniques présentées postérieurement au dépôt de la demande et ne figurant pas dans le présent fascicule.

# (54) Dérivés pyréthrinoides comportant un hétérocycle azoté, leurs procédé et intermédiaires de préparation, leur application comme pesticides et les compositions les renfermant.

(30) Priorité: **20.07.87 FR 8710202**

(43) Date de publication de la demande:
**25.01.89 Bulletin 89/04**

(45) Mention de la délivrance du brevet:
**10.03.93 Bulletin 93/10**

(84) Etats contractants désignés:
**CH DE ES FR GB GR IT LI NL**

(56) Documents cités:
**EP-A- 0 050 534**
**EP-A- 0 064 353**
**EP-A- 0 176 387**

**CHEMIE DER PFLANZENSCHTUTZ- UND SCHAEDLINGSBEKAEMPFUNGSMITTEL, vol. 7, 1981, pages 7-9, Springer Verlag Berlin**

(73) Titulaire: **ROUSSEL-UCLAF**
**35, Boulevard des Invalides**
**F-75007 Paris(FR)**

(72) Inventeur: **Demassey, Jacques**
**Allée Saint Jacques Chalifert**
**F-77144 Montevrain(FR)**
Inventeur: **Demoute, Jean-Pierre**
**249 bis, rue de Rosny**
**F-93100 Montreuil-Sous-Bois(FR)**
Inventeur: **Tessier, Jean**
**30, rue Jean Moulin**
**F-94300 Vincennes(FR)**

(74) Mandataire: **Tonnellier, Marie-José et al**
**111, route de Noisy B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 300 898 B1

Rank Xerox (UK) Business Services
(3.10/3.5x/3.0.1)

## Description

EP-A-50 534 et EP-A-176 387 concernent des dérivés pyréthrinoïdes utilisables comme pesticides.

La présente invention concerne de nouveaux dérivés pyréthrinoïdes comportant un hétérocycle azoté, leurs procédé et intermédiaires de préparation, leur application comme pesticides et les compositions les renfermant.

L'invention a pour objet les composés de formule (I) :

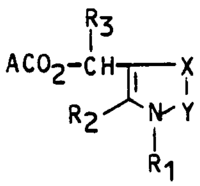

(I)

dans laquelle X représente un atome de soufre ou d'oxygène, Y représente un radical C = O, C = S ou $CH_2$, $R_1$ représente un atome d'hydrogène, un atome d'halogène, un radical alcoyle linéaire, ramifié ou cyclique, saturé ou insaturé, renfermant jusqu'à 8 atomes de carbone, éventuellement substitué par un ou plusieurs halogènes, ou un radical aryle renfermant jusqu'à 14 atomes de carbone, $R_2$ représente un atome d'hydrogène, un radical alcoyle linéaire, ramifié ou cyclique saturé ou insaturé, renfermant jusqu'à 8 atomes de carbone, éventuellement susbstitué par un ou plusieurs halogènes, un radical aryle renfermant jusqu'à 14 atomes de carbone, un radical $CF_3$, $NO_2$, C≡N, un atome d'halogène, un radical alcoxyle renfermant jusqu'à 8 atomes de carbone, $R_3$ représente un atome d'hydrogène, un radical alcoyle renfermant de 1 à 3 atomes de carbone, un radical C≡N ou un radical C≡CH et A représente le reste d'un acide pyréthrinoïde $ACO_2H$, dans lequel

- ou bien A représente un radical

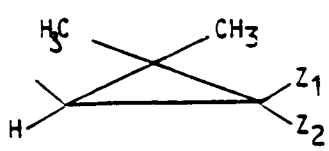

dans lequel :

- $Z_1$ et $Z_2$ représentent chacun un radical méthyle,
- ou $Z_1$ représente un atome d'hydrogène et
- soit $Z_2$ représente un radical

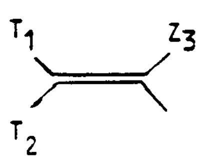

dans lequel $Z_3$ représente un atome d'hydrogène ou d'halogène et ou bien $T_1$ et $T_2$, identiques ou différents, représentent un atome d'hydrogène, un atome d'halogène, un radical alcoxyle ou alcoyle renfermant de 1 à 8 atomes de carbone, un radical $CF_3$, CN ou phényle éventuellement substitué par un halogène ou bien $T_1$ et $T_2$ forment ensemble un radical cycloalcoyle renfermant de 3 à 6 atomes de carbone ou un radical :

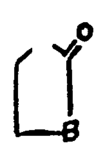

dans lequel B représente un atome d'oxygène ou de soufre ;

- soit $Z_2$ représente un radical :

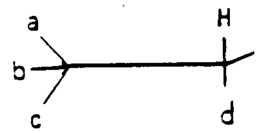

dans lequel a, b, c et d, identiques ou différents, représentent chacun un atome d'halogène,

- soit $Z_2$ représente un radical :

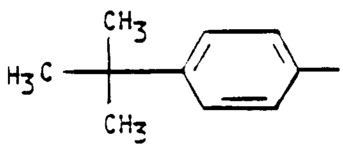

- soit $Z_2$ représente un radical :

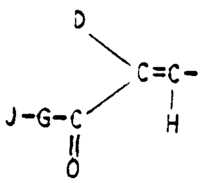

dans lequel D représente un atome d'hydrogène ou d'halogène, un radical alcoxyle renfermant de 1 à 8 atomes de carbone, G représente un atome d'oxygène ou de soufre et J représente ou bien un radical alcoyle linéaire, ramifié ou cyclique, saturé ou insaturé, renfermant de 1 à 8 atomes de carbone, éventuellement substitué par un ou plusieurs halogènes ou par des groupements fonctionnels identiques ou différents, ou bien un groupement phényle ou bien un radical pyridyle, furyle, thiényle, oxazolyle ou thiazolyle,

- ou bien A représente un radical :

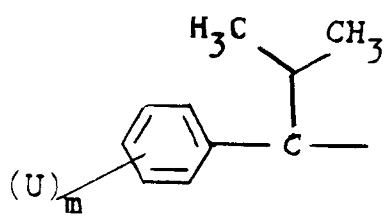

dans lequel U, en position quelconque sur le noyau benzénique, représente un atome d'halogène, un radical alcoyle renfermant de 1 à 8 atomes de carbone ou un radical alcoxy renfermant de 1 à 8 atomes de carbone, éventuellement substitués par 1 ou plusieurs atomes d'halogènes, m représente le nombre 0, 1 ou 2 et quand m est 2, les substituants peuvent être identiques ou différents,

- ou bien A représente un radical :

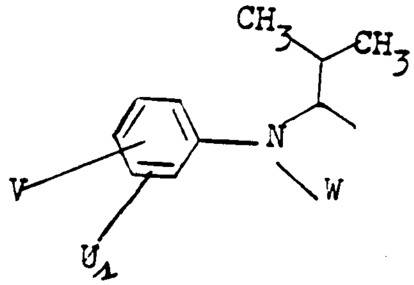

dans lequel $U_1$ et V identiques ou différents, représentent un atome d'hydrogène, un atome d'halogène ou un radical trifluorométhyle, W représente un atome d'hydrogène ou un radical méthyle, sous toutes leurs formes stéréoisomères possibles, ainsi que les mélanges de ces stéréoisomères.

Lorsque $R_1$ ou $R_2$ représente un radical alcoyle, il s'agit de préférence d'un radical méthyle, éthyle, n-propyle, isopropyle, cyclopropyle, cyclopropylméthyle, butyle, isobutyle, terbutyle, cyclobutyle, n-pentyle, cyclopentyle, n-hexyle ou cyclohexyle ou encore d'un radical allyle, propargyle ou butynyle. Lorsque $R_1$ ou $R_2$ représente un radical aryle, il s'agit de préférence du radical phényle.

Lorsque $R_2$ représente un atome d'halogène, il s'agit de préférence d'un atome de fluor, de chlore ou de brome.

Lorsque $R_3$ représente un radical alcoyle, il s'agit de préférence du radical méthyle.

L'invention a particulièrement pour objet les composés de formule (I) dans lesquels Y représente un radical C=O.

L'invention a plus particulièrement pour objet :

- les composés de formule (I) dans lesquels $R_3$ représente un atome d'hydrogène ;
- les composés de formule (I) dans lesquels $R_1$ représente un radical alcoyle insaturé renfermant jusqu'à 4 atomes de carbone, comme par exemple le radical 2-propynyle ou le radical 2-propényle ;
- les composés de formule (I) dans lesquels $R_2$ est le radical $CF_3$.

Lorsque $T_1$, $T_2$ ou $Z_3$ représente un atome d'halogène, il s'agit de préférence d'un atome de fluor, de chlore ou de brome.

Lorsque $T_1$ ou $T_2$ représente un radical alcoyle ou alcoxyle, il s'agit de préférence du radical méthyle, éthyle, n-propyle, méthoxy, éthoxy ou n-propoxy.

a, b, c et d représentent de préférence un atome de chlore ou de brome.

Lorsque D représente un atome d'halogène, il s'agit de préférence d'un atome de fluor, de chlore ou de brome.

Lorsque J représente un radical alcoyle substitué par un ou plusieurs groupements fonctionnels, on entend par alcoyle un radical renfermant de 1 à 8 atomes de carbone comme, par exemple, le radical méthyle, éthyle, propyle, isopropyle, n-butyle, isobutyle ou terbutyle et par groupement fonctionnel les radicaux cyano, $OR_a$ dans lequel $R_a$ représente un atome d'hydrogène ou un radical alcoyle linéaire ou ramifié comportant de 1 à 8 atomes de carbone,

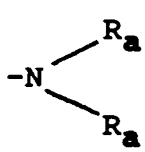

dans lequel $R_a$ est tel que défini ci-dessus, les deux radicaux $R_a$ pouvant être identiques ou différents ou 2-tétrahydropyrranyloxy

Lorsque J représente un radical alcoyle substitué par un ou plusieurs groupements fonctionnels, on peut citer comme valeurs préférées de J, les radicaux :

$-(CH_2)n_1-CHal_3$ dans lequel $n_1$ est un entier de 1 à 8 et Hal un atome d'halogène, par exemple le radical $-CH_2-CCl_3$, $-CH_2-CF_3$,

$-CH_2-CH_2-CCl_3$ ou $CH_2-CH_2-CF_3$ ;

$-(CH_2)n_2-CH(Hal)_2$ dans lequel Hal est défini comme ci-dessus et $n_2$ est un nombre de 0 à 8, par exemple le radical $-CH_2-CHCl_2$,

$-CH_2-CHF_2$ ou $-CHF_2$ ;

$-(CH_2)n_1-CH_2$ Hal dans lequel $n_1$ et Hal sont définis comme ci-dessus, par exemple le radical $-CH_2-CH_2Cl$ ou $-CH_2-CH_2F$,

$-C-(CHal_3)_3$ dans lequel Hal est défini comme ci-dessus, par exemple le radical $-C-(Cf_3)_3$ ou

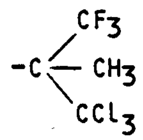

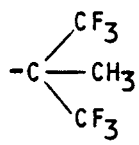

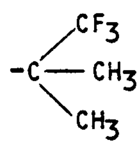

ou 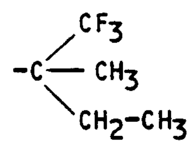

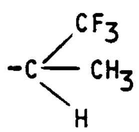 ou 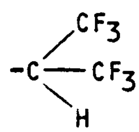

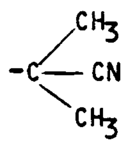

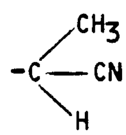

ou $-(CH_2)_n-CN$

dans lequel n est défini comme précédemment,

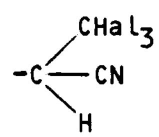

dans lequel Hal est défini comme précédemment,
par exemple le radical

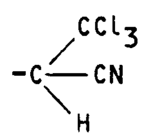

$-(CH_2)n_1-OR_a$, dans lequel $n_1$ est défini comme précédemment et $R_a$ représente un atome d'hydrogène ou un radical alcoyle linéaire ou ramifié, comportant de 1 à 8 atomes de carbone, par exemple le radical
$- CH_2-O-CH_3$, $-CH_2-CH_2-O-CH_3$, $-CH_2-CH_2-O-CH_2-CH_3$ ou
$-CH_2-CH_2-OH$ ;
$-(CH_2)n_1$

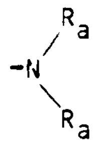

dans lequel $n_1$ et $R_a$ sont définis comme précédemment et les deux radicaux $R_a$ peuvent être différents entre eux, par exemple le radical

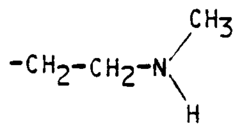

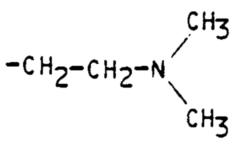 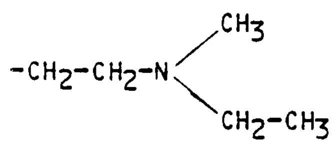 ;

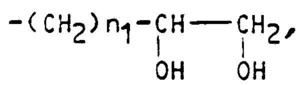

dans lequel $n_1$ est défini comme précédemment par exemple le radical

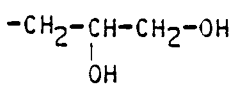 ; 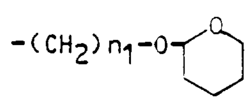 ,

dans lequel $n_1$ est défini comme précédemment, par exemple le radical

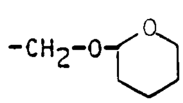 ou 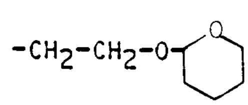 .

L'invention a plus spécialement pour objet

- les composés de formule I dans lesquels A représente le radical

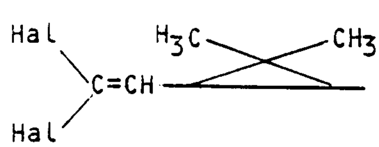

sous toutes les formes stéréoisomères possibles, ou sous forme de mélanges de ces stéréoisomères, Hal représentant un atome de fluor, de brome, de chlore ou d'iode et tout particulièrement ceux dans lesquels Hal représente un atome de brome ;

- les composés de formule I dans lesquels A représente un radical

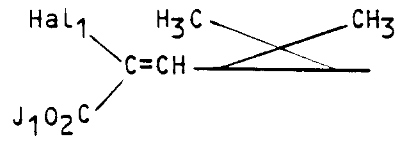

sous toutes les formes stéréoisomères possibles, ou sous forme de mélanges de ces stéréoisomères, dans lequel $Hal_1$ représente un atome d'halogène et $J_1$ représente un radical alcoyle saturé linéaire, ramifié ou cyclique, renfermant de 1 à 8 atomes de carbone, éventuellement substitué par un ou plusieurs halogènes, la double liaison ayant la géométrie E, par exemple ceux dans lesquels $Hal_1$ représente un atome de fluor ;
- les composés de formule I dans lesquels A représente le radical

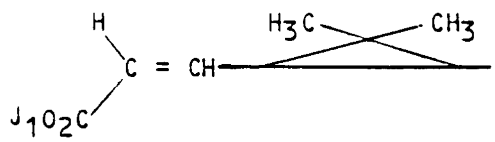

sous toutes les formes stéréoisomères possibles, ou sous forme de mélanges de ces stéréoisomères, dans lequel $J_1$ est défini comme précédemment, la double liaison ayant la géométrie (Z) et parmi ceux-ci les composés dans lesquels $J_1$ représente un radical terbutyle ou encore ceux dans lesquels $J_1$ représente un radical

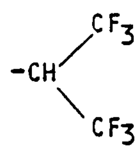

- les composés de formule (I) dans lesquels A représente un radical

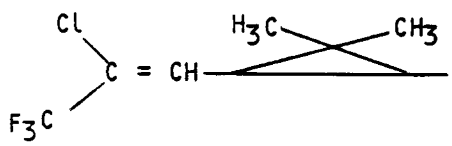

sous toutes ses formes stéréoisomères possibles, ou sous forme de mélanges de ces stéréoisomères.

L'invention a plus particulièrement pour objet les composés dont la préparation est donnée plus loin dans la partie expérimentale et notamment les produits des exemples 7, 8, 11, 23 et 25 ou encore ceux des exemples 1, 2, 3, 4, 18 ou 22.

L'invention a également pour objet un procédé de préparation des composés de formule (I) caractérisé en ce que l'on soumet un alcool de formule II) :

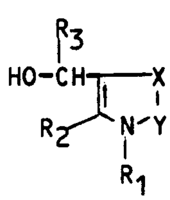

(II)

dans laquelle X, Y, $R_1$, $R_2$ et $R_3$ conservent la même signification que précédemment, à l'action d'un acide de formule (III) :

$ACO_2$ (III)

dans laquelle A conserve la même signification que précédemment ou d'un dérivé fonctionnel de cet acide.

Le dérivé fonctionnel d'acide utilisé est de préférence un chlorure d'acide.

Lorsque l'on fait réagir l'acide de formule (III) et l'alcool de formule (II), on opère de préférence en présence de dicyclohexylcarbodi-imide.

Les alcools de formule (II) sont des produits nouveaux et sont en tant que tels un des objets de la présente invention.

L'invention a plus particulièrement pour objet les composés de formule (II) décrits dans les préparations figurant ci-après, dans la partie expérimentale.

Les alcools peuvent être préparés selon le schéma réactionnel suivant :

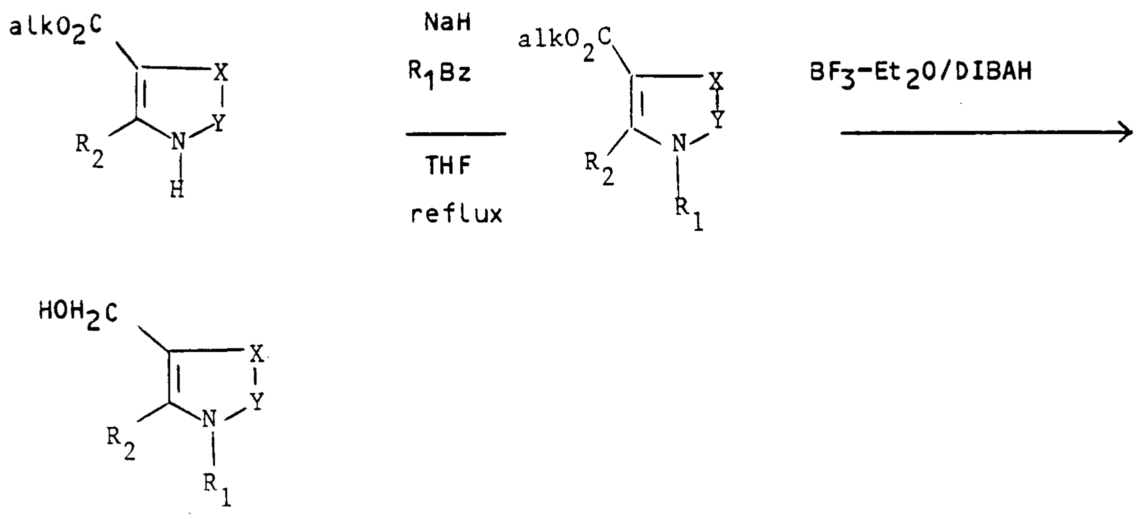


La préparation de certains alcools de formule II est donnée ci-après dans la partie expérimentale.

Les composés de formule

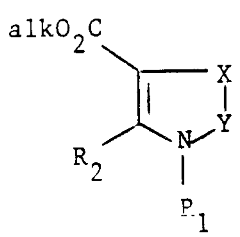

utilisés comme produits de départ sont décrits par exemple dans

CA 92 110 998 P
95 62 179 K
97 182 393 P
101 23 466 Z

ou, dans le brevet européen EP 027020.

Les composés de formule (I) présentent d'intéressantes propriétés qui permettent leur utilisation dans la lutte contre les parasites ; il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des végétaux, les parasites des locaux et les parasites des animaux à sang chaud.

Les produits de formule (I) peuvent être utilisés notamment pour lutter contre les insectes dans le domaine agricole, pour lutter par exemple contre les pucerons, les larves de lépidoptères et les coléoptères. Ils sont utilisés à des doses comprises entre 10g et 300g de matière active à l'hectare.

Les produits de formule (I) peuvent également être utilisés pour lutter contre les insectes dans les locaux, pour lutter notamment contre les mouches, les moustiques et les blattes.

Les composés de formule (I) présentent notamment un très bon pouvoir de knock down.

Il ressort des résultats de tests biologiques ci-après que les produits de formule (I) possèdent également une remarquable activité aphicide.

Les produits de formule (I) peuvent aussi être utilisés pour lutter contre les acariens parasites des végétaux.

Les composés de formule (I) présentent en effet de remarquables propriétés acaricides comme le montrent les résultats de tests biologiques ci-après.

Les composés de formule (I) peuvent aussi être utilisés pour lutter contre les nématodes parasites des végetaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des végétaux, les parasites des locaux et les parasites des animaux à sang chaud, caractérisées en ce qu'elles renferment au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

L'invention a notamment pour objet les compositions insecticides renfermant comme principe actif au moins l'un des produits définis ci-dessus.

Les compositions insecticides selon l'invention contiennent de préférence de 0,005% à 10% en poids de matière active.

Selon un mode opératoire avantageux, pour un usage dans les locaux, les compositions insecticides selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles de Machilus Thumbergii), poudre de tige de pyrèthre, poudre de feuille de cèdre, poudre de bois (telle que la sciure de pin) amidon et poudre de coque de noix de coco. La dose de matière active peut alors être, par exemple, de 0,03 à 1% en poids.

Dans le cas où l'on utilise un support fibreux incombustible, la dose de matière active peut alors être, par exemple, de 0,03 à 95% en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95% en poids.

L'invention a également pour objet, les compositions acaricides renfermant comme principe actif au moins un des produits de formule (I) définis ci-dessus.

L'invention a également pour objet les compositions nématicides renfermant comme principe actif au moins un des produits de formule (I) ci-dessus.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions,

émulsions, solutions.

Pour l'usage acaricide, on utilise de préférence des poudres mouillables, pour pulvérisation foliaire, contenant de 1 à 80% ou des liquides pour pulvérisation foliaire contenant de 1 à 500 g/l de principe actif. On peut également employer des poudres pour poudrage foliaires contenant de 0,05 à 3% de matière active.

Pour l'usage nématicide, on utilise de préférence des liquides pour traitement des sols contenant de 300 à 500g/l de principe actif.

Les composés acaricides et nématicides selon l'invention sont utilisés, de préférence, à des doses comprises entre 1 et 100g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des anti-oxydants.

L'invention a donc également pour objet les compositions destinées à l'alimentation animale, renfermant comme principe actif au moins l'un des produits de formule (I) telle que définie précédemment.

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3,4,5,6-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcool-alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones d'alcool 3,4,5,6-tetrahydrophtalimido-méthylique, d'alcool 5-benzyl 3-furyl méthylique, d'alcool3-phénoxy benzylique et d'alcools alpha-cyano 3-phénoxy benzyliques des acides 2,2-diméthyl 3-(1,2,2,2-tétrahalo éthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les associations selon l'invention présente notamment l'intérêt soit de permettre de combattre, par la polyvalence de leur action, une gamme de parasites, plus étendue, soit de manifester, dans certains cas, un effet de synergie.

Pour exalter l'activité biologique des produits de l'invention, on peut les additionner à des synergies classiques utilisés en pareil cas tel le 1-(2,5,8-trioxadodécyl) 2-propyl 4,5-méthylènedioxy benzène (ou butoxyde de pipéronyle) ou la N-(2-éthyl heptyl) bicyclo [2.2.1] 5-heptène-2,3-dicarboximide, ou le pipéronyl-bis-2(2'-n-butoxy éthoxy) éthylacétal (ou tropital).

L'invention a donc pour objet les compositions pesticides définies précédemment, caractérisées en ce qu'elles renferment en outre un synergiste des pyréthrinoïdes.

Les exemples suivants illustrent l'invention sans toutefois la limiter.

**Exemple 1 :** [1R [1alpha , 3alpha, (E)]] 2,2-diméthyl 3-[2-fluoro 3-oxo 3-(1,1-diméthyl éthoxy) propényl] cyclopropane carboxylate de [2-oxo 3-(2-propynyl) 2,3-dihydro 4-trifluorométhyl thiazol-5-yl] méthyle.

On ajoute à 0 ± 5¤C, 0,12 g de diméthylaminopyridine et 0,78 g de dicyclohexylcarbodiimide dans une solution renfermant 0,7 g d'acide [1R [ 1alpha, 3alpha, (E)]] 2,2-diméthyl 3-[2-fluoro 3-oxo 3-(1,1-diméthyl éthoxy) propényl] cyclopropanecarboxylique et 0,94 g de 5-hydroxyméthyl 3-(2-propynyl) 4-trifluorométhyl 2-(3H)-thiazolone et 15 cm3 de chlorure de méthylène. On laisse revenir à la température ambiante et agite pendant une heure. On filtre, concentre le filtrat sous pression réduite à 40¤C. on chromatographie le résidu obtenu sur silice en éluant par le mélange hexane-acétate d'éthyle (85-15). On obtient ainsi 1,1 g du

produit recherché.

alpha$_D$ = + 38¤,5 ± 2¤ C = 0,5% $CHI_3$

En opérant comme à l'exemple 1 à partir des acides et des alcools correspondants, on a obtenu les produits suivants :

**Exemple 2 :** [1R [1alpha, 3alpha, (E)]] 2,2-diméthyl 3-(2-fluoro 3-oxo 3-éthoxy propényl) cyclopropanecarboxylate de [2-oxo 3-(2-propynyl) 4-trifluorométhyl 2,3-dihydro thiazol-5-yl] méthyle.

alpha$_D$ = + 36¤ ± 2¤ C = 0,5% $CHCl_3$.

**Exemple 3 :** [1R (1alpha, 3alpha)] 2,2-diméthyl 3-(2,2-dibromoéthényl) cyclopropane carboxylate de [2-oxo 3-(2-propynyl) 4-trifluorométhyl 2,3-dihydrothiazol-5-yl] méthyle.

F = 68¤C alpha$_D$ = + 14¤± 1¤ C = 1% $CHCl_3$.

**Exemple 4 :** [1R [1alpha, 3alpha, (Z)]] 2,2-diméthyl 3-[3-oxo 3-(1,1-diméthyléthoxy) 1-propényl] cyclopropane carboxylate de [2-oxo 3-(2-propényle) 4-trifluorométhyl 2,3-dihydrothiazol-5-yl] méthyle.

alpha$_D$ = + 67¤5 ± 2,5¤ C = 0,65% $CHCl_3$.

**Exemple 5 :** 2,2,3,3-tétraméthyl cyclopropane carboxylate de [2-oxo 3-(2-propynyl) 4 -trifluorométhyl 2,3-dihydrothiazol-5-yl] méthyle. F< 50¤C.

**Exemple 6 :** [1S] [1-(4-chlorophényl) 2-méthyl propyl] carboxylate de [2-oxo 3-(2-propynyl) 4-trifluorométhyl 2,3-dihydrothiazol-5-yl] méthyle.

alpha$_D$ = + 5¤ ± 2¤ C = 0,85% toluène.

**Exemple 7 :** [1R, [1alpha, 3alpha,(Z)]] 2,2-diméthyl 3-[2-chloro 3,3,3-trifluoro propényl] cyclopropanecarboxylate de [2-oxo 3-(2-propynyl) 4-trifluorométhyl 2,3-dihydrothiazol-5-yl] méthyle.

alpha$_D$ = + 35¤ ± 2¤5 C = 0,5% $CHCl_3$.

**Exemple 8:** [1R, [1alpha, 3alpha, (Z)]] 2,2-diméthyl 3-[3-oxo 3-(1,1,1,3,3,3-hexafluoro 2-propyloxy) 1-propényl] cyclopropane carboxylate de [2-oxo 3-(2-propynyl) 4-trifluorométhyl 2,3-dihydrothiazol-5-yl] méthyle.

alpha$_D$ = + 33¤ ± 2¤ C = 0,5% $CHCl_3$

**Exemple 9 :** [1R [1alpha, 3alpha (Z)]] 2,2-diméthyl 3-[2-chloro 2-(4-chlorophényl) éthényl] cyclopropanecarboxylate de [2-oxo 3-(2-propynyl) 4-trifluorométhyl 2,3-dihydrothiazol-5-yl] méthyle.

alpha$_D$ = + 46¤ ± 1,5¤ C = 0,9% $CHCl_3$

**Préparation I : 5-hydroxyméthyl 3-(2-propynyl) 4-trifluorométhyl 2 (3H) thiazolone.**

**Stade A :** 2,3-dihydro 2-oxo 3-(2-propynyl) 4-trifluorométhyl 5-thiazol carboxylate d'éthyle.

On dissout 6,9 g de 2,3-dihydro 2-oxo 4-trifluorométhyl 5-thiazole carboxylate d'éthyle (préparé selon le procédé décrit dans CA 92 110 998P) dans 70 cm3 de tétrahydrofuranne à 0¤ ± 5¤C. On ajoute alors 1,44g d'hydrure de sodium à 50% dans l'huile. Une fois le dégagement gazeux terminé, on ajoute 15 cm3 de 3-bromopropyne. On porte ensuite le mélange réactionnel au reflux. On maintient le mélange réactionnel au reflux pendant 48 heures. On verse le mélange réactionnel sur une solution glacée de phosphate monosodique. On extrait au chlorure de méthylène, on sèche, filtre et concentre sous pression réduite. On chromatographie le résidu obtenu en éluant par le mélange hexane-acétate d'éthyle (85-15). On obtient ainsi 5,57 g du produit recherché.

**Stade B :** 5-hydroxyméthyl 3-(2-propynyl) 4-trifluorométhyl 2 (3H) thiazolone (A) et 2,3 dihydro 3-(2-propynyl) 4-trifluoro méthyl 5-thiazol méthanol (B).

On ajoute à -65¤C, 1,9 cm3 d'éthérate de trifluorure de bore dans une solution renfermant 3,84 g de produit préparé au stade A dans 40 cm3 de toluène. On agite le mélange réactionnel pendant 30 minutes et introduit en 90 minutes environ à -65¤C ± 3¤ C 69 cm3 d'hydrure de diisobutyl aluminium en solution 1,2 molaire dans le toluène et agite encore 1 heure à -70¤C. On verse dans une solution molaire glacée de tartrate double de potassium et de sodium. On agite pendant 45 minutes le mélange réactionnel, décante, extrait à l'acétate d'éthyle, lave àl'eau, puis avec une solution aqueuse saturée en chlorure de sodium. On sèche et amène à sec, on obtient ainsi 2,7g de résine que l'on chromatographie sur silice en éluant par le mélange hexane-éther isopropylique (1-1). On isole ainsi 1,85g de produit recherché (A)rf = 0,1 et 0,20 g du produit recherché (B) rf = 0,2.

**Exemple 10 :** [1R, [1alpha, 3alpha, (E)]] 2,2-diméthyl 3-[3-éthoxy 2-fluoro 3-oxo propényl] cyclopropanecarboxylate de [2,3-dihydro 2-oxo 3-(2-propynyl) 4-trifluorométhyl oxazol-5-yl)] méthyle.

$alpha_D$ = + 13¤5 ± 2¤ C = 0,5% $CHCl_3$.

**Exemple 11 :** [1R [1alpha, 3alpha, (E)]] 2,2-diméthyl 3-[3-(1,1-diméthyl éthoxy) 2-fluoro 3-oxo propényl] cyclopropane carboxylate de [2,3-dihydro 2-oxo 3-(2-propynyl) 4-trifluorométhyl oxazol-5-yl] méthyle.

$alpha_D$ = + 25¤ ± 2¤ C = 0,6% $CHCl_3$.

**Exemple 12 :** [1R (1alpha, 3alpha)] 2,2-diméthyl 3-(2,2-dibromoéthényl) cyclopropane carboxylate de [2,3-dihydro 2-oxo 3-(2-propynyl) 4-trifluorométhyl oxazol-5-yl] méthyle.

F = 74¤C $alpha_D$ = -6¤ ± 1¤C C = 0,9% $CHCl_3$.

**Préparation 2 : 5-hydroxyméthyl 3-(2-propynyl) 4-trifluorométhyl 2-(3H)-oxazolone.**

**Stade A :** 2,3-dihydro 2-oxo 3-(2-propynyl) 4-trifluorométhyl oxazol-5-carboxylate d'éthyle.

On ajoute à 10¤C 5,4 cm3 d'azodicarboxylate de diéthyle dans une solution renfermant 7 g de 2,3-dihydro 2-oxo 4-trifluorométhyl oxazol-5-carboxylate d'éthyle (préparé comme il est indiqué dans le brevet EP 027020) 70 cm3 de tétrahydrofuranne, 8,15 g de triphénylphosphine et 3,3 cm3 d'alcool propargylique. On agite pendant 1 heure à 20¤C. On verse dans l'eau, extrait à l'acétate d'éthyle, sèche, filtre et concentre. On chromatographie le produit obtenu sur silice en éluant par le mélange hexane-acétate d'éthyle 85-15. On obtient ainsi 4,6 g de produit recherché fondant à 72¤C.

**Stade B :** 5-hydroxyméthyl 3-(2-propynyl) 4-trifluorométhyl 2-(3H)-oxozalone

On introduit à -30¤C, 520 mg d'hydrure d'aluminium et de lithium dans une solution renfermant 3,6g du produit préparé au stade A dans 7,5 cm3 d'éther éthylique. On verse sur une solution de tartrate double de sodium et de potassium et maintient le mélange réactionnel sous agitation pendant 16h. On décante, extrait à l'acétate d'éthyle, sèche, filtre et concentre. On chromatographie le produit sur silice en éluant par le mélange hexane-acétate d'éthyle (7-3). On obtient ainsi 0,96g de produit fondant à 64¤C.

**Exemple 13 :** [1R (1alpha, 3alpha,)] 2,2-diméthyl 3-(2,2-dibromoéthényl) cyclopropane carboxylate de (2,3-dihydro 3-cyclopropylméthyl 2-oxo 4-trifluorométhyl thiazol-5-yl) méthyle

$alpha_D$ = + 6¤ ± 2¤ C = 0,6% $CHCl_3$

**Exemple 14 :** [1R,[1alpha, 3alpha, ( E)]] 2,2-diméthyl 3-(3-éthoxy 2-fluoro 3-oxo propényl) cyclopropane carboxylate de (2,3-dihydro 3-cyclopropyl méthyl 2-oxo 4-trifluorométhyl thiazol-5-yl) methyle.

$alpha_D$ = + 33¤ ± 2¤ C = 0,6% $CHCl_3$

**Exemple 15 :** [1R [1alpha, 3alpha, (E)]] 2,2-diméthyl 3-[3-(1,1-diméthyléthoxy) 2-fluoro 3-oxo propényl] cyclopropane carboxylate de (3-cyclopropylméthyl 2,3-dihydro 2-oxo 4-trifluorométhyl thiazol-5-yl) méthyle.

$alpha_D$ = + 30¤5 ± 2¤ C = 0,5% $CHCl_3$.

**Préparation 3 : 3-cyclopropylméthyl 5-hydroxyméthyl 4-trifluorométhyl 2-(3H)-thiazolone.**

**Stade A :** 2,3-dihydro 3-cyclopropylméthyl 2-oxo 4-trifluorométhyl thiazol-5-carboxylate d'éthyle.

On introduit à 0¤ ± 5¤ 200 mg d'hydrure de sodium à 50% dans l'huile, dans une solution renfermant 1 g de 2,3-dihydro 2-oxo 4-trifluorométhyl 5-thiazole carboxylate d'éthyle [CA 92 110 998P] et 10 cm3 de tétrahydrofuranne. On agite pendant une demi-heure à la température ambiante. On ajoute ensuite 1 cm3 de bromométhyl cyclopropane et 0,63 g d'iodure de sodium. On agite 1 heure à la température ambiante et 1 heure à 80¤C. On laisse revenir à la température ambiante, dilue au chlorure de méthylène, verse sur une solution de phosphate acide de sodium, extrait au chlorure de méthylène, sèche, filtre et concentre. On chromatographie le résidu obtenu sur silice en éluant par le mélange hexane-acétate d'éthyle (9-1). On obtient 1,03g de produit recherché, rf = 0,25.

**Stade B :** 3-cyclopropylméthyl 5-hydroxyméthyl 4-trifluorométhyl 2-(3H)-thiazolone.

On ajoute à -65¤C, 1,9 cm3 d'éthérate de trifluorure de bore dans une solution renfermant 4,05g de produit obtenu comme au stade A dans 40 cm3 de toluène. On agite pendant une demi heure à -65¤C et introduit 70 cm3 d'une solution 0,5M d'hydrure de diisobutyl aluminium dans le toluène. On verse le mélange réactionnel sur 400 cm3 d'une solution aqueuse molaire de tartrate double de sodium et de potassium et agite pendant 1 heure. On décante, extrait à l'acétate d'éthyle, lave à l'eau et sèche. On filtre et concentre pour obtenir 3,5g d'un produit que l'on chromatographie sur silice en éluant par le mélange hexane-acétate d'éthyle (7-3). On obtient 2,87 g du produit recherché, rf = 0,25.

**Exemple 16 :** [1R (1alpha, 3alpha)] 2,2-diméthyl 3-(2,2-dibromoéthényl) cyclopropane carboxylate de [2,3-dihydro 2-oxo 3-(2-propényl) 4-trifluorométhyl thiazol-5-yl] méthyle.

F<50¤C. $alpha_D$ = + 7¤ ± 1¤5 C = 0,7% $CHCl_3$

**Exemple 17 :** [1R[1alpha, 3alpha, (E)]] 2,2-diméthyl 3-[3-éthoxy 2-fluoro 3-oxo propényl] cyclopropane carboxylate de [2,3-dihydro 2-oxo 3-(2-propényl) 4-trifluorométhyl thiazol-5-yl] méthyle.

$alpha_D$ = + 37¤5 ± 1¤5 C = 0,7% $CHCl_3$.

**Exemple 18 :** [IR[1alpha, 3alpha, (E)]] 2,2-diméthyl 3-[3-(1,1-diméthyl éthoxy) 2-fluoro 3-oxo propényl] cyclopropane carboxylate de [2,3-dihydro 2-oxo 3-(2-propényl) 4-trifluorométhyl thiazol-5-yl] méthyle.

$alpha_D$ = + 49¤ ± 1¤5 C = 0,8% $CHCl_3$.

**Préparation 4 : 5-hydroxyméthyl 3-(2-propényl) 4-trifluorométhyl 2-(3H)-thiazolone**

**Stade A :** 2,3-dihydro 2-oxo 3-(2-propényl) 4-trifluorométhyl thiazol 5-carboxylate d'éthyle.

On chauffe à 90¤C pendant 3 heures, 10 g de 2,3-dihydro 2-oxo 4-trifluorométhyl-5-thiazol carboxylate d'éthyle (CA 92 110 998 P) 200 cm3 de toluène, 2,6 g de potasse en pastilles 6 cm3 de bromure d'allyle, 2,8g de bromure de tétrabutylammonium. On laisse revenir à la température ambiante en agitant pendant 2 heures. On lave à l'aide d'une solution normale de soude, à l'aide d'une solution normale d'acide chlorhydrique, à l'eau, avec une solution saturée de chlorure de sodium. On sèche, filtre et concentre. On chromatographie le résidu sur silice en éluant par le mélange hexane-acétate d'éthyle (9-1). On obtient ainsi 8,65g du produit recherché rf = 0,25.

**Stade B:** 5-hydroxyméthyl 3-(2-propényl) 4-trifluorométhyl 2-(3H)-thiazolone.

On introduit à -65¤C, 4,3 cm3 d'éthérate de trifluorure de bore dans une solution renfermant 8,65 g de produit obtenu au stade A dans 100 cm3 de toluène. On agite le mélange réactionnel à -65¤C, puis introduit 155 cm3 de solution 0,5M d'hydrure de diisobutyl aluminium dans le toluène. On verse dans une solution molaire glacée de tartrate double de sodium et de potassium et agite pendant 2 heures le mélange ainsi obtenu. On décante, extrait à l'acétate d'éthyle, lave à l'eau, avec une solution saturée de chlorure de sodium, sèche, filtre et concentre. On chromatographie le produit obtenu sur silice en éluant par le mélange hexane-acétate d'éthyle (7-3). On obtient ainsi 4 g de produit recherché rf = 0,25.

**Exemple 19 :** [1R(1alpha, 3alpha, (E))] 2,2-diméthyl 3-(2-fluoro 3-oxo 3-éthoxy 1-propényl) cyclopropane carboxylate de [2-oxo 3-phényl 4-trifluorométhyl 2,3-dihydrothiazol-5-yl] méthyle.

$alpha_D$ = + 41¤ ± 1¤ C = 1,2% $CHCl_3$.)

**Exemple 20 :** [1R(1alpha, 3alpha (E))] 2,2-diméthyl 3-[2-fluoro 3-oxo 3-(1,1-diméthyléthoxy) 1-propényl] cyclopropane carboxylate de [2-oxo 3-phényl (4-trifluorométhyl 2,3-dihydrothiazol-5-yl] méthyle.

$alpha_D$ = +48¤ ± 2¤ C = 0,5% $CHCl_3$

**Préparation 5 : 3-phényl 4-trifluorométhyl 5-hydroxyméthyl 2-(3H)-thiazolone.**

**Stade A :** [2-oxo 3-phényl 4-hydroxy 4-trifluorométhyl 2,3,4,5-tétrahydro thiazol-5-yl] carboxylate d'éthyle.

On dissout 12,2 g de N-phénylthiocarbamate d'éthyle (Ber (1916) 49 1027) et 14,7 g de trifluoroacétyl alpha-chloroacétate d'éthyle dans 300 cm3 de toluène. On porte au reflux sous barbotage d'azote pendant 24 heures le mélange réactionnel, on laisse refroidir et distille à sec. On récupère 22 g de produit recherché.

**Stade B :** [2-oxo 3-phényl 4-trifluorométhyl 2,3-dihydrothiazol-5-yl] carboxylate d'éthyle.

On dissout le produit obtenu au stade précédent dans 150 cc de chlorure de méthylène. On refroidit à 0-5¤C et ajoute, sous agitation et atmosphère d'azote, une solution renfermant 11 cm3 de chlorure de méthane sulfonyle et 40 cm3 de chlorure de méthylène, puis en 30 minutes une solution de 18 cm3 de triéthylamine dans 30 cm3 de chlorure de méthylène. On laisse le mélange réactionnel revenir à la température ambiante en agitant pendant 18 heures. On verse sur un mélange d'eau et de glace, on décante, extrait au chlorure de méthylène, lave, sèche et amène à sec. On obtient 27 g de résidu que l'on chromatographie sur silice en éluant dans le mélange hexane-acétate d'éthyl (9-1). On obtient 4,49 g fondant à 55¤C.

**Stade C :** 3-phényl 4-trifluorométhyl 5-hydroxyméthyl 2-(3H)-thiazolone.

On ajoute à -65¤C, 0,5 cm3 d'éthérate de trifluorure de bore dans une solution renfermant 1 g du produit préparé au stade précédent dans 20 cm3 de toluène. On maintient sous agitation pendant 30 minutes et ajoute 20 cm3 d'unesolution 1,2M d'hydrure de diisobutyl aluminiumdans le toluène On verse dans un mélange d'une solution de tartrate de sodium et potassium de glace. On maintient le mélange sous agitation pendant 1 heure, décante, extrait à l'acétate d'éthyle, lave à l'eau, sèche et amène à 1 sec. On chromatographie le résidu sur silice en éluant par le mélange hexane-acétate d'éthyle (7-3) et on obtient ainsi 0,36g de produit recherché rf = 0,15. F = 135¤C

**Exemple 21 :** [1R(1alpha, 3alpha)] 2,2-diméthyl 3-(2,2-dibromoéthényl) cyclopropane carboxylate de [2-oxo 3-(2-propynyl) 2,3-dihydrothiazol-5-yl] méthyle.

F = 107¤-108¤C $alpha_D$ = + 14,5¤ ± 0,5¤ C = 1,4% $CHCl_3$.

**Exemple 22 :** [1R[1alpha, 3alpha (E)] 2,2-diméthyl 3-(2-fluoro 3-oxo 3-éthoxy 1-propényl) cyclopropane carboxylate de [2-oxo 3-(2-propynyl) 2,3-dihydro thiazol-5-yl] méthyle.

$alpha_D$ = + 47¤ ± 1,5¤ (C = 0,8% $CHCl_3$).

**Exemple 23 :** [1R [(1alpha, 3alpha) (E)]] 2,2-diméthyl 3-[2-fluoro 3-oxo 3-(2,2-diméthyl éthoxy) 1-propényl] cyclopropane carboxylate de [2-oxo 3-(2-propynyl) 2,3-dihydrothiazol-5-yl] méthyle.

$alpha_D$ = + 52¤ ± 2¤ (C = 0,5% $CHCl_3$).

**Préparation 6 : 3-(2-propynyl) 5-hydroxyméthyl 2-(3H)-thiazolone.**

**Stade A :** (2-oxo 2,3-dihydrothiazol-5-yl) carboxylate d'éthyle.

On ajoute 50 cm3 de dichloréthane dans un mélange renfermant 8,25g de formyl chloroacétate d'éthyle, et 5,25 g de thiocarbamate d'éthyle. On chauffe le mélange réactionnel au reflux durant 4 heures sous barbotage d'azote. On évapore à sec, on dissout le résidu obtenu dans 5 cm3 d'éther isopropylique tiède. On amorce la cristallisation et laisse 16 heures environ à + 5¤C. On essore, lave et sèche, on obtient ainsi 2,44 g de produit recherché fondant à 127¤C.

**Stade B :** [2,3-dihydro 2-oxo 3-(2-propynyl) thiazol-5-yl] carboxylate d'éthyle.

On chauffe à 95¤C et agite pendant 3 heures un mélange de 8,65 g de produit préparé au stade précédent, dans 130 cm3 de toluène, 2,8 g de potasse 4,5 cm3 de 3-bromo 1-propyne,1,66 g de bromure de tétrabutyl ammonium. On décante, on lave à la soude N, à l'acide chlorhydrique N, à l'eau saturée en chlorure de sodium, sèche et amène à sec. On chromatographie le résidu obtenu en éluant par le mélange hexane-chloroforme-acétone(70-15-15). On isole ainsi 4,74 g de produit recherché fondant à 70¤-71¤C.

**Stade C :** 3-(2-propynyl) 5-hydroxyméthyl 2-(3H)-thiazolone.

On refroidit à -65¤C--70¤C une solution de 8,44 g de produit préparé au stade précédent dans 100 cm3 de toluène. On ajoute 5,6 g d'éthérate de trifluorure de bore, agite pendant trente minutes le mélange réactionnel et introduit en 2 h 15, 200 cm3 d'une solution 1,2M d'hydrure de disobutyl aluminium dans le toluène. On agite encore pendant trente minutes. On verse dans 1,5 l de solution M de tartrate de potassium et de sodium. On agite pendant 1 heure, décante, extrait à l'acétate d'éthyle, lave à l'eau saturée en chlorure de sodium, sèche et amène à sec. On chromatographie le résidu obtenu en éluant par le mélange hexane-chloroforme-acétone(6-2-2). On isole ainsi 2,33 g de produit fondant à 97¤-98¤C.

**Exemple 24 :** [1R, (1alpha, 3alpha)] 2,2-diméthyl 3-(2,2-dibromoéthényl) cyclopropane carboxylate de [2-oxo 3-(2-propynyl) 4-méthyl 2,3-dihydro-thiazol-5-yl] méthyle.

$alpha_D$ = -2¤ ± 2¤ C = 0,5% $CHCl_3$.

**Exemple 25 :** [1R, [1alpha, 3alpha] (E)]] 2,2-diméthyl 3-(2-fluoro 3-oxo 3-éthoxy propényl) cyclopropane carboxylate de [2-oxo 3-(2-propynyl) 4-méthyl 2,3-dihydro thiazol-5-yl] méthyle.

$alpha_D$ = + 38¤± 2¤ C = 0,55 % $CHCl_3$)

**Example 26 :** [1R, [1alpha, 3alpha, (E)]] 2,2-diméthyl 3-[2-fluoro 3-oxo 3-(1,1-diméthyléthoxy) 1-propényl] cyclopropane carboxylate de [2-oxo 3-(2-propynyl) 4-méthyl 2,3-dihydro thiazol-5-yl] méthyle.

$alpha_D$ = + 46¤ ± 2¤ C = 0,6% $CHCl_3$.

**Préparation 7 : 3-(2-propynyl) 4-méthyl 5-hydroxyméthyl 2-(3H)-thiazolone**

**Stade A :** [2,3-dihydro 2-oxo 4-méthylthiazol-5-yl] carboxylate d'éthyle.

On refroidit à 5¤C une solution renfermant 19,4g de bêta-amino crotonate d'éthyle et 40 cm5 de chlorobenzène. On introduit en 20 minutes, 23,2 g de chlorure de chlorothio formyle. On laisse revenir le mélange réactionnel à la température ambiante, le maintient à cette température pendant 1 heure puis porte pendant 4 heures à 80¤C. On refroidit, filtre, lave à l'éther de pétrole et sèche. On obtient ainsi 19,9 g du produit recherché fondant à 180¤C après recristallisation dans l'éthanol.

**Stade B :** [2,3-dihydro 2-oxo 3-(2-propynyl) 4-méthyl thiazol-5-yl] carboxylate d'éthyle.

On agite sous atomosphère inerte 20,6 g de produit obtenu au stade A dans 300 cm3 de toluène, 6,2 g de potasse en pastilles, 10,4 cm3 de 3-bromo 1-propyne et 3,65 g de bromure de tétrabutylammonium. On chauffe à 95¤C pendant 19 heures puis laisse revenir à température ambiante. On décante, lave la phase organique à la soude N, à l'acide chlorhydrique à l'eau, puis à l'eau saturée en chlorure de sodium, sèche et amène à sec. On chromatographie le résidu sur silice, élue par un mélange hexane-chloroforme-acétone (8-1-1) et obtient 16,4 g de produit attendu F = 65¤C.

**Stade C :** 3-(2-propynyl) 4-méthyl 5-hydroxyméthyl 2-(3H)-thiazolone.

On opère comme au stade C de la préparation 6 à partir de 2,25 g de produit obtenu ci-dessus et obtient 1,03 g de produit attendu F = 62¤C.

**Exemple 27 :** [1R (1alpha, 3alpha)] 2,2-diméthyl 3-(2,2-dibromo éthényl) cyclopropane carboxylate de [2-oxo 3-phénylméthyl 4-trifluorométhyl 2,3-dihydro thiazol-5-yl] méthyle.

F = 70¤C alpha$_D$ = + 7,5¤ 1¤ C = 0,85% $CHCl_3$.

**Exemple 28 :** [1R,[1alpha, 3alpha, (E)]] 2,2-diméthyl 3-(2-fluoro 3-oxo 3-éthoxy 1-propényl) cyclopropane carboxylate de [2-oxo 3-phényl méthyl 4-trifluoro méthyl 2,3-dihydrothiazol-5-yl] méthyle. rf = 0,22 silice éluant (hexane-éther isopropylique (1-1).

alpha$_D$ = + 37 ± 1¤ C = 1% $CHCl_3$

**Exemple 29 :** [1R, [1alpha, 3alpha (E)]] 2,2-diméthyl-3-[(2-fluoro 3-oxo 3-(1,1-diméthyléthoxy) 1-propényl)] cyclopropane carboxylate de [2-oxo 3-phénylméthyl 4-trifluoro méthyl 2,3-dihydro thiazol 5-yl] méthyle.

alpha$_D$ = + 41¤ ± 2¤ C = 0,7% $CHCl_3$.

**Préparation 8 : 3-phénylméthyl 4-trifluorométhyl 5-hydroxyméthyl 2-(3H)-thiazolone.**

**Stade A :** [2,3-dihydro 2-oxo 3-phénylméthyl 4-trifluorométhylthiazol 5-yl] carboxylate d'étyle.

On chauffe à 70¤C pendant 7 heures, 5 g de (2,3-dihydro 2-oxo 4-trifluorométhyl thiazol-5-yl) carboxylate d'éthyle (CA 92 110 998 P), 80 cm3 de toluène, 1,16 g de potasse, 3,7 cm3 de bromure de benzyle, 1,61 g de bromure de tétrabutylammonium. On laisse revenir à la température ambiante, décante, lave et sèche. On obtient 7,2 g de produit que l'on chromatographie sur silice en éluant par le mélange hexane-éther isopropylique (8-2). On isole ainsi 5,46 g de produit recherché rf = 0,17.

**Stade B :** 3-phénylméthyl 4-trifluorométhyl 5-hydroxyméthyl 2-(3H)-thiazolone.

On agite pendant 30 minutes à -65¤-70¤C, 3,13 g de produit préparé au stade A, 30 cc de toluène et 1,3 cm3 d'éthérate de trifluorure de bore. On ajoute en 2 heures 48 cm3 de solution 1,2 M d'hydrure de diisobutyl aluminium dans le toluène. On verse le produit obtenu dans une solution molaire glacée de tartrate de sodium et de potassium. On agite pendant 1 heure, on décante, extrait à l'acétate d'éthyle, lave, sèche et amène à sec. On chromatographie sur silice le produit obtenu en éluant par le mélange hexane-éther isopropylique (1,1). On obtient 2,16 g de produit recherché rf = 0,1.

**Exemple 30 :** [1R[1alpha, 3alpha, (E)]] 2,2-diméthyl 3-[2-fluoro 3-oxo 3-éthoxy 1-propényl] cyclopropanecarboxylate de (RS) [2-oxo 3-(2-propynyl) 4-trifluorométhyl 2,3-dihydrothiazol-5-yl] prop-2-ynyle

$alpha_D$ = + 29¤5 ± 2¤   C = 0,6% $CHCl_3$

**Exemple 31 :** [1R, [1alpha, 3alpha (E)] 2,2-diméthyl 3-[2-fluoro 3-oxo 3-(1,1-diméthyl éthoxy) 1-propényl] cyclopropane carboxylate de (RS) [2-oxo 3-(2-propynyl) 4-trifluorométhyl 2,3-dihydro thiazol-5-yl)] prop-2-ynyle.

$alpha_D$ = + 38 ± 2¤5   C = 0,5% $CHCl_3$.

**Préparation 9 : 3-(2-propynyl) 4-trifluorométhyl 5-(1-hydroxy prop-2-ynyl) 2-(3H)-thiazolone.**

**Stade A :** [2-oxo 3-(2-propynyl) 4-trifluorométhyl 2,3-dihydrothiazol-5-yl] carboxaldéhyde.

On agite pendant 7 h 30 un mélange réactionnel renfermant 4 g de 2,3-dihydro 2-oxo 3-(2-propynyl) 4-trifluorométhyl-5-thiazole méthanol (voir préparation 1), 60 cm3 de chlorure de méthylène et 8 g de bioxyde de manganèse. On agite pendant 29 heures le mélange réactionnel. On filtre, rince au chlorure de méthylène et amène à sec le filtrat. On chromatographie le résidu sur silice en éluant par le mélange hexane-éther isopropylique (1-1). On obtient ainsi 3,48 g de produit recherché fondant à 58¤-59¤C.

**Stade B :** 3-(2-propynyl) 4-trifluorométhyl 5-(1-hydroxy prop-2-ynyl) 2-(3H)-thiazolone.

On ajoute une solution de 1,17 g de produit préparé au stade précédent 7,5 cm3 de tétrahydrofuranne dans 18,75 cc d'une solution 0,8M de bromure d'éthynyl magnésium dans le tétrahydrofuranne. On agite le mélange réactionnel pendant 1 heure à 0/5¤C. On verse sur une solution de phosphate monosodique, on décante, extrait à l'acétate d'éthyle, lave à l'eau, à l'eau saturée en chlorure de sodium et amène à sec. On chromatographie le résidu obtenu sur silice en éluant par le mélange hexane-éther isopropylique (1-1). On obtient 1,13 g de produit recherché rf = 0,15.

**Exemple 32 :** [1R[1alpha, 3alpha (E)]] 2,2-diméthyl 3-[2-fluoro 3-oxo 3-(1,1-dimethyléthoxy) 1-propényl] cyclopropane carboxylate de (RS) [2-oxo 3-(2-propynyl) 4-trifluorométhyl 2,3-dihydrothiazol-5-yl] éthyle.

$alpha_D$ = 53 ± 2¤5   C = 0,5% $CHCl_3$.

**Exemple 33 :** [1R[1alpha, 3alpha (E)]] 2,2-diméthyl 3-(2-fluoro 3-oxo 3-éthoxy 1-propényl) cyclopropane carboxylate de (RS) [2-oxo 3-(2-propynyl) 4-trifluoro méthyl 2,3-dihydrothiazol-5-yl] éthyle.

$alpha_D$ = + 45¤ ± 2¤   C = 0,7% $CHCl_3$.

**Préparation 10 : (RS) 3-(2-propynyl) 4-trifluorométhyl 5-(1-hydroxyéthyl) 2-(3H)-thiazolone.**

On mélange à 0¤C, 30 cm3 d'une solution 1M d'iodure de méthyl magnésium dans l'éther éthylique, 2,35 g de [2-oxo 3-(2-propynyl) 4-trifluorométhyl 2,3-dihydro thiazole-5-yl] carboxaldéhyde et 12 cm3 de tétrahydrofuranne. On maintient la suspension sous agitation pendant 40 minutes. On ajoute une solution aqueuse saturée de phosphate monosodique, verse dans un mélange d'eau, de glace et d'éther éthylique. On décante, extrait à l'éther éthylique, lave, sèche et amène à sec. On obtient 2,7 g de résidu que l'on chromatographie sur silice en éluant par le mélange hexane-éther isopropylique (7-3). On obtient ainsi 1,87 g de produit recherché. F = 77¤-78¤C.

**Exemple 34 :** [1R, [1alpha, 3alpha, (E)]] 2,2-diméthyl 3-[3-éthoxy 2-fluoro 3-oxo 1-propényl] cyclopropane carboxylate de [3-(2-propynyl) 4-trifluorométhyl 2,3-dihydro thiazol-5-yl] méthyle.

$alpha_D$ = + 25¤ ± 2¤   C = 0,5% toluène.

**Exemple 35 :** [1R-[1alpha, 3alpha (E)]] 2,2-diméthyl 3-[2-fluoro 3-(1,1-diméthyl éthoxy) 3-oxo 1-propényl] cyclopropane carboxylate de [3-(2-propynyl) 4-trifluorométhyl 2,3-dihydro thiazol-5-yl] méthyle.

On chromatographie sur silice en éluant par le mélange hexane-acétate d'éthyle 8,2. rf = 0,35.
L'alcool de départ des 2 produits précédents, à savoir le 2,3-dihydro 3(2-propynyl) 4-trifluorométhyl-5-thiazole méthanol est le produit 8 dont la préparation est donnée au stade B de la préparation 1.

**Exemple 36 :** [1R-[1alpha, 3alpha, (Z)]] 3-(2-cyano 2-méthoxyéthényl) 2,2-diméthyl cyclopropane carboxylate de [2-oxo 3-(2-propynyl) 4-trifluorométhyl 2,3-dihydrothiazol-5-yl] méthyle.

$alpha_D$ = + 31¤ ± 2¤ c = U,7% toluène.

**Exemple 38 :** [1R, (1alpha, 3alpha)] 3-(2,2-dibromoéthényl) 2,2-diméthyl cyclopropane carboxylate de [3-(2-butynyl) 2-oxo 4-trifluorométhyl 2,3-dihydrothiazol-5-yl] méthyle.

$alpha_D$ = + 10¤ ± 2¤ c = 0,5% toluène.

**Exemple 39 :** [1R, 1alpha, 3alpha, (E)] 2,2-diméthyl 3-[2-fluoro 3-(1,1-diméthyléthoxy) 3-oxo 1-propényl] cyclopropane carboxylate de [3-(2-butynyl) 2-oxo 4-trifluorométhyl 2,3-dihydrothiazol-5-yl] méthyle.

$alpha_D$ = + 49¤ ± 2,5¤ c = 0,5% toluène.

**Exemple 40 :** [1R, 1alpha, 3alpha, (E)] 2,2-diméthyl 3-[3-éthoxy 2-fluoro 3-oxo 1 -propényl] cyclopropane carboxylate de [3-(2-butynyl) 2-oxo 4-trifluorométhyl 2,3-dihydrothiazol-5-yl] méthyle.

$alpha_D$ = + 49,5¤ ± 2,5¤ c = 0,6% toluène.

**Préparation 11 : Alcool [2-oxo 3-(2-butynyl) 4-trifluorométhyl 2,3- dihydro-thiazol-5-yl] methylique.**

**Stade A :** [3-(2-butynyl) 2-oxo 4-trifluoromethyl 2,3-dihydrothiazol-5-yl] carboxylate d'éthyle.

On mélange 6,54 g de 2,3-dihydro 2-oxo 4-trifluorométhyl 5-thiazole carboxylate d'éthyle, 150 cm3 de tétrahydrofuranne, 6,52 g de triphénylphosphine et 3,5 cm3 d'alcool butyn-2-ol-1. On ajoute en 1 heure à +10¤C, +15¤C, 6,15 cm3 d'azodicarboxylate de diéthyle dans 50 cm3 de tétrahydrofuranne. On agite pendant 20 heures à 20¤C, verse sur de la glace, extrait à l'acétate d'éthyle, sèche la phase organique, l'évapore à sec, chromatographie le résidu sur silice en éluant au mélange flugène trichlorotrifluoroéthane-oxyde de diisopropyle (8-2) et obtient 3,96 g de produit attendu.
Spectre IR ($CHCl_3$) :
absorption à 1738 et 1685cm$^{-1}$ (C=O), 1590cm$^{-1}$ (C=C) et 2245cm$^{-1}$ (C=C).

**Stade B :** Alcool [2-oxo 3-(2-butynyl) 4-trifluorométhyl 2,3-dihydrothiazol-5-yl] méthylique.

On mélange 35 cm3 d'hydrure de diisobutylaluminium (1,2M dans le toluène), 7 cm3 de toluène et 16 cm3 d'hexane, refroidit à -5¤C,0¤C et ajoute en 15 minutes 26 cm3 de butyllithium à 15% dans l'hexane. On agite à 0¤C pendant 1 heure, puis refroidit à -65¤C. On ajoute en 20 minutes à 54 cm3 de la solution obtenue ci-dessus, 3,9 g de produit obtenu au stade A dans 25 cm3 de toluène. On agite pendant 3 heures à -65¤C puis ajoute en 10 minutes 0,35 g de boro-hydrure de sodium, en solution dans 25 cm3 d'éthanol. On agite pendant 45 minutes en laissant remonter la température, verse sur 100g de glace et 100 cm3 d'une solution aqueuse 1M de tartrate double de sodium et de potassium. On agite 1 heure, extrait à l'éther éthylique, sèche la phase organique et concentre à sec. On obtient 3,3 g de produit attendu.
Spectre 1R ($CHCl_3$) :
absorption à 1672 cm$^{-1}$ (C=O), 3613 cm$^{-1}$ (-OH) et 2240 cm$^{-1}$ (C=C).

**Exemple 41** ::[1R, 1alpha, 3alpha, (E)] 2,2-diméthyl 3-[2-fluoro 3-(1,1-diméthyléthoxy) 3-oxo 1-propényl] cyclopropane carboxylate de [3-(3,3-dichloro 2-propényl) 2-oxo 4-trifluorométhyl 2,3-dihydrothiazol-5-yl|] méthyle.

$alpha_D$ = + 55,5¤ ± 2¤ c = 0,7% toluène.

**Exemple 42 :** [1R, 1alpha, 3alpha, (E)] 2,2-diméthyl 3-(3-éthoxy 2-fluoro 3-oxo 1-propényl] cyclopropane carboxylate de [3-(3,3-dichloro 2-propényl) 2-oxo 4-trifluorométhyl 2,3-dihydrothiazol-5-yl] méthyle.

$alpha_D$ = +53,5¤ ± 2,5¤   c = 0,5% toluène.

**Exemple 43 :** [1R, (1alpha, 3alpha)] 3-(2,2-dibromoéthényl) 2,2-diméthyl cyclopropane carboxylate de [3-(3,3-dichloro 2-propényl) 2-oxo 4-trifluorométhyl 2,3-dihydrothiazol-5-yl] méthyle.
$alpha_D$ = + 23,5¤ ± 1,5¤   c = 1% toluène. F = 66¤C.

**Préparation 12 : Alcool [3-(3,3-dichloro 2-propényl) 2-oxo 4-trifluorométhyl 2,3-dihydrothiazol-5-yl] méthylique.**

Stade A : [3-(3,3-dichloro 2-propényl) 2-oxo 4-trifluorométhyl 2,3-dihydrothiazol-5-yl] carboxylate d'éthyle.

On mélange 6 g de 2,3-dihydro 2-oxo 4-trifluorométhyl 5-thiazole carboxylate d'éthyle et 30 cm3 de diméthylformamide, ajoute lentement 1,27 g d'hydrure de sodium (à 50% dans l'huile), agite pendant 15 minutes puis introduit à +5¤C en 10 minutes une solution de 5,78 g de 1,1-dichloro 3-bromo 1-propène dans 10 cm3 de diméthylformamide. On agite pendant 45 minutes à +5¤C puis à 20¤C pendant 67 heures. On verse dans une solution aqueuse de phosphate monosodique, extrait à l'éther isopropylique, sèche la phase organique, évapore le solvant et chromatographie le résidu sur silice en éluant au mélange hexane-acétate d'éthyle (9-1). On obtient 8,7 g de produit attendu.
Spectre IR ($CHCl_3$) :
absorption à 1738 et 1685 $cm^{-1}$ (C=O), 1597 et 1628 $cm^{-1}$ (C=C).

**Stade B :** Alcool [3-(3,3-dichloro 2-propényl) 2-oxo 4-trifluorométhyl 2,3-dihydrothiazol-5-yl] méthylique.

On opère de manière analogue à celle décrite au stade B de la préparation 11, en utilisant au départ 4,35 g de produit obtenu au stade A. On obtient 3,61 g de produit attendu. F = 81¤C.

**Exemple 44 :** préparation d'un concentré soluble

On effectue un mélange homogène de :

| | |
|---|---|
| Produit de l'exemple 2 | 0,25 g |
| Butoxyde de pipéronyle | 1,00 g |
| Tween 80® | 0,25 g |
| Topanol A® | 0,1 g |
| Eau | 98,4 g |

**Exemple 45 :** préparation d'un concentré émulsifiable

On mélange intimement :

| | |
|---|---|
| Produit de l'exemple 3 | 0,015 g |
| Butoxyde de pipéronyle | 0,5 g |
| Topanol A® | 0,1 g |
| Tween 80® | 3,5 g |
| Xylène | 95,885 g |

**Exemple 46 :** préparation d'un concentré émulsifiable

On effectue un mélange homogène de :

| | |
|---|---|
| Produit de l'exemple 11 | 1,5 g |
| Tween 80® | 20,00 g |
| Topanol A® | 0,1 g |
| Xylène | 78,4 g |

**Exemple 47 :** préparation d'une composition fumigène

On mélange d'une façon homogène :

| | |
|---|---|
| Produit de l'exemple 10 | 0,25 g |
| Poudre de tabu | 25,00 g |
| Poudre de feuille de cèdre | 40,00 g |
| Poudre de bois de pin | 33,75 g |
| Vert brillant | 0,5 g |
| p-nitrophénol | 0,5 g |

**ETUDE BIOLOGIOUE**

1) Etude de l'activité de choc sur mouche domestique

Les insectes tests sont des mouches domestiques femelles âgées de 4 jours. On opère par pulvérisation directe en chambre de Kearns et March en utilisant comme solvant un mélange d'acétone (5%) et d'Isopar L®(solvant pétrolier) (quantité de solvant utilisée 2 ml en une seconde. On utilise 50 insectes par traitement. On effectue les contrôles toutes les minutes jusqu'à 10 minutes, puis à 15 minutes et l'on détermine le KT 50 par les méthodes habituelles.

Les résultats obtenus sont les suivants :

| Composé de l'exemple | KT 50 (en mn) concentration à 0,1 g/l |
|---|---|
| 1 | 5,5 |
| 2 | 2,25 |
| 3 | 2,45 |
| 7 | 3,5 |
| 10 | 3,1 |
| 11 | 2,6 |
| 17 | 2,9 |
| 18 | 7,6 |
| 22 | 2,14 |
| 23 | 1,52 |
| 24 | 5,6 |
| 25 | 1,16 |
| 34 | 5,7 |
| 36 | 4,88 |

**Conclusion :** Les produits de l'invention sont doués d'un très bon effet de choc sur mouches domestiques.

2) Activité sur Tétranychus urticae. Essai adulticide.

On utilise des plants de haricot comportant deux feuilles cotylédonaires. Ces plants sont traités au pistolet Fisher avec une solution acétonique du produit. Après séchage, 25 femelles de l'acarien Tétranychus urticae sont disposées par feuilles soit 50 individus par dose expérimentée par plant. Le contrôle d'efficacité est effectué après 80 heures de contact. On mesure la CL 50 en mg/hl.

| Exemple | CL 50 |
|---|---|
| 1 | 75 |
| 4 | 44 |
| 7 | 31 |
| 8 | 30 |
| 11 | 29 |
| 18 | 75 |
| 30 | 96 |

**Conclusion :** les produits de l'invention sont doués d'un remarquable effet acaricide sur Tétranychus urticae.

3) Etude de l'effet létal sur Aphis cracivora.

On utilise des adultes après 7 jours et l'on emploie 10 Aphis par concentration utilisée. On utilise une méthode de contact-injection. On effectue le traitement au pistolet de Fisher d'une feuille de fève que l'on dépose dans une boîte de Pétri en matière plastique sur une rondelle de papier humidifiée. Le traitement est effectué à l'aide de 2 ml de solution acétonique de produit à tester (1 ml par face de feuille). L'infestation par insecte est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Composé | CL 50 en mg/litre |
|---|---|
| 1 | 1,98 |
| 3 | 1,3 |
| 7 | 0,8 |
| 8 | 0,35 |
| 18 | 0,95 |

**Revendications**
**Revendications pour les Etats contractants suivants : CH, DE, FR, GB, IT, LI, NL**

**1.** L'invention a pour objet les composés de formule (I) :

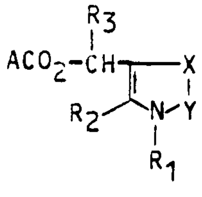

(I)

dans laquelle X représente un atome de soufre ou d'oxygène, Y représente un radical C = O, C = S ou $CH_2$, $R_1$ représente un atome d'hydrogène, un atome d'halogène, un radical alcoyle linéaire, ramifié ou cyclique, saturé ou insaturé, renfermant jusqu'à 8 atomes de carbone, éventuellement substitué par un ou plusieurs halogènes, ou un radical aryle renfermant jusqu'à 14 atomes de carbone, $R_2$ représente

un atome d'hydrogène, un radical alcoyle linéaire, ramifié ou cyclique saturé ou insaturé, renfermant jusqu'à 8 atomes de carbone, éventuellement substitué par un ou plusieurs halogènes, un radical aryle renfermant jusqu'à 14 atomes de carbone, un radical $CF_3$, $NO_2$, C≡N, un atome d'halogène, un radical alcoxyle renfermant jusqu'à 8 atomes de carbone, $R_3$ représente un atome d'hydrogène, un radical alcoyle renfermant de 1 à 3 atomes de carbone, un radical C≡N ou un radical C≡CH et A représente le reste d'un acide pyréthrinoïde $ACO_2H$ dans lequel

- ou bien A représente un radical

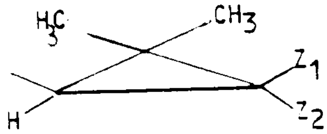

dans lequel :

- $Z_1$ et $Z_2$ représentent chacun un radical méthyle,
- ou $Z_1$ représente un atome d'hydrogène et
- soit $Z_2$ représente un radical

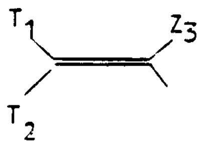

dans lequel $Z_3$ représente un atome d'hydrogène ou d'halogène et ou bien $T_1$ et $T_2$, identiques ou différents, représentent un atome d'hydrogène, un atome d'halogène, un radical alcoxyle ou alcoyle renfermant de 1 à 8 atomes de carbone, un radical $CF_3$, CN ou phényle éventuellement substitué par un halogène ou bien $T_1$ et $T_2$ forment ensemble un radical cycloalcoyle renfermant de 3 à 6 atomes de carbone ou un radical :

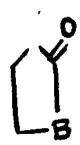

dans lequel B représente un atome d'oxygène ou de soufre ;

- soit $Z_2$ représente un radical :

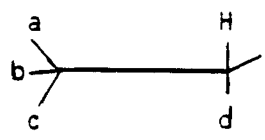

dans lequel a, b, c et d, identiques ou différents, représentent chacun un atome d'halogène,

- soit $Z_2$ représente un radical :

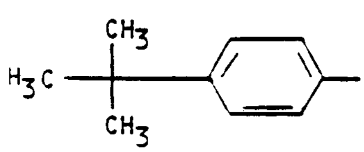

- soit $Z_2$ représente un radical :

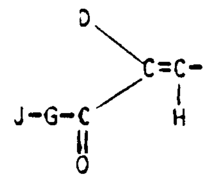

dans lequel D représente un atome d'hydrogène ou d'halogène, un radical alcoxyle renfermant de 1 à 8 atomes de carbone, G représente un atome d'oxygène ou de soufre et J représente ou bien un radical alcoyle linéaire, ramifié ou cyclique, saturé ou insaturé, renfermant de 1 à 8 atomes de carbone, éventuellement substitué par un ou plusieurs halogènes ou par des groupements fonctionnels identiques ou différents, choisis parmi les radicaux cyano, $OR_a$ dans lequel $R_a$ représente un atome d'hydrogène ou un radical alcoyle linéaire ou ramifié comportant de 1 à 8 atomes de carbone,

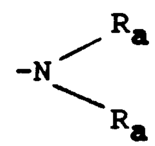

dans lequel $R_a$ est tel que défini ci-dessous, les deux radicaux $R_a$ pouvant être identique ou différents ou 2-tétrahydropyrranyloxy, ou bien un groupement phényle ou bien un radical pyridyle, furyle, thiényle, oxazolyle ou thiazolyle,

- ou bien A représente un radical :

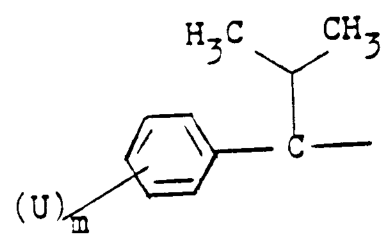

dans lequel U, en position quelconque sur le noyau benzénique, représente un atome d'halogène, un radical alcoyle renfermant de 1 à 8 atomes de carbone ou un radical alcoxy renfermant de 1 à 8 atomes de carbone, éventuellement substitués par 1 ou plusieurs atomes d'halogènes, m représente le nombre 0, 1 ou 2 et quand m est 2, les substituants peuvent être identiques ou différents,

- ou bien A représente un radical :

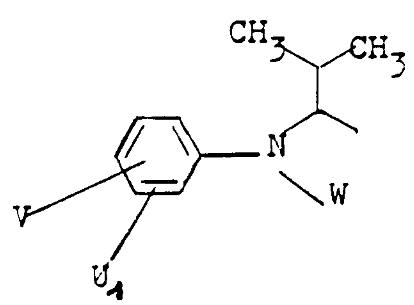

dans lequel $U_1$ et V identiques ou différents, représentent un atome d'hydrogène, un atome d'halogène ou un radical trifluorométhyle, W représente un atome d'hydrogène ou un radical

méthyle, sous toutes leurs formes stéréoisomères possibles, ainsi que les mélanges de ces stéréoisomères.

2. Les composés de formule (I) tels que définis à la revendication 1 dans lesquels Y représente un radical C=O.

3. Les composés de formule (I) tels que définis à la revendication 1 ou 2 dans lesquels $R_3$ représente un atome d'hydrogène.

4. Les composés de formule (I) tels que définis à la revendication 1, 2 ou 3 dans lesquels $R_1$ représente un radical alcoyle insaturé renfermant jusqu'à 4 atomes de carbone.

5. Les composés de formule (I) tels que définis à la revendication 4 dans lesquels $R_1$ est le radical 2-propynyle.

6. Les composés de formule (I) tels que définis à la revendication 4 dans lesquels $R_1$ est le radical 2-propényle.

7. Les composés de formule (I) tels que définis à l'une quelconque des revendications 1 à 6 dans lesquels $R_2$ est le radical $CF_3$.

8. Les composés de formule (I) tels que définis à l'une quelconque des revendications 1 à 7 dans lesquels A représente le radical :

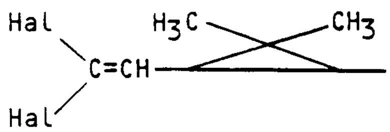

sous toutes les formes stéréoisomères possibles, ou sous forme de mélanges de ces stéréoisomères, Hal représentant un atome de fluor, de brome, de chlore ou d'iode.

9. Les composés de formule (I) tels que définis à la revendication 8, dans lesquels Hal représente un atome de brome.

10. Les composés de formule (I) tels que définis à l'une quelconque des revendications 1 à 7, dans lesquels A représente un radical :

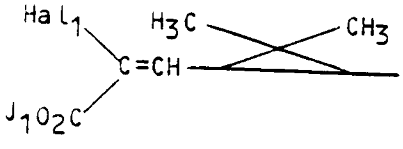

sous toutes les formes stéréoisomères possibles, ou sous forme de mélanges de ces stéréoisomères, dans lequel $Hal_1$ représente un atome d'halogène et $J_1$ représente un radical alcoyle saturé linéaire, ramifié ou cyclique, renfermant de 1 à 8 atomes de carbone, éventuellement substitué par un ou plusieurs halogènes, la double liaison ayant la géométrie E.

11. Les composés de formule (I) tels que définis à la revendication 10, dans lesquels $Hal_1$ représente un atome de fluor.

12. Les composés de formule (I) tels que définis à l'une quelconque des revendications 1 à 7 dans lesquels A représente le radical :

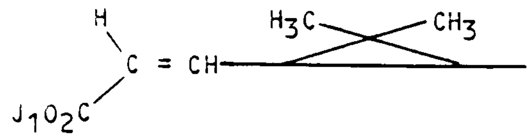

sous toutes les formes stéréoisomères possibles, ou sous forme de mélanges de ces stéréoisomères, dans lequel $J_1$ est défini comme la revendication 10, la double liaison ayant la géométrie Z.

**13.** Les composés de formule (I) tels que définis à la revendication 12, dans lesquels $J_1$ représente un radical terbutyle.

**14.** Les composés de formule (I) tels que définis à la revendication 12 dans lesquels $J_1$ représente un radical :

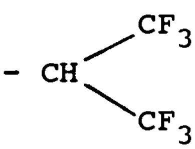

**15.** Les composés de formule (I) tels que définis à l'une quelconque des revendications 1 à 7 dans lesquels A représente un radical :

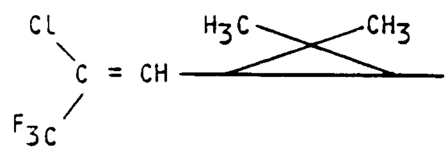

sous toutes ses formes stéréoisomères possibles, ou sous forme de mélanges de ces stéréoisomères.

**16.** Les composés de formule (I) tels que définis à la revendication 1, dont les noms suivent :

- le [1R [1alpha, 3alpha, (Z)]] 2,2-diméthyl 3-[2-chloro 3,3,3-trifluoropropényl] cyclopropane carboxylate de (2-oxo 3-(2-propynyl) 4-trifluorométhyl 2,3-dihydrothiazol-5-yl)] méthyle ;
- le [1R [1alpha, 3alpha, (Z)]] 2,2-diméthyl 3-[3-oxo 3-(1,1,1,3,3,3-hexafluoro 2-propyloxy] 1-propényl] cyclopropane carboxylate de (2-oxo 3-(2-propynyl) 4-trifluorométhyl 2,3-dihydrothiazol-5-yl)] méthyle ;
- le [1R [1alpha, 3alpha, (E)]] 2,2-diméthyl 3-[3-oxo 3-(1,1-diméthyléthoxy) 2-fluoropropényl] cyclopropane carboxylate de (2-oxo 3-(2-propynyl) 4-trifluorométhyl 2,3-dihydrooxazol-5-yl)] méthyle ;
- le [1R [1alpha, 3alpha, (E)]] 2,2-diméthyl 3-[2-fluoro 3-oxo 3-(2,2-diméthyléthoxy) 1-propényl] cyclopropane carboxylate de (2-oxo 3-(2-propynyl) 2,3-dihydrothiazol-5-yl)] méthyle ;
- et le [1R [1alpha, 3alpha, (E)]] 2,2-diméthyl 3-[2-fluoro 3-oxo 3-éthoxypropényl) cyclopropane carboxylate de (2-oxo 3-(2-propynyl) 4-méthyl 2,3-dihydrothiazol-5-yl)] méthyle.

**17.** Procédé de préparation des composés de formule (I) tels que définis à l'une quelconque des revendications 1 à 16, caractérisé en ce que l'on soumet un alcool de formule (II) :

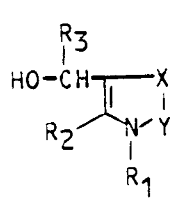

(II)

dans laquelle X, Y, $R_1$, $R_2$ et $R_3$ conservent la même signification que dans la revendication 1, à l'action d'un acide de formule (III) :

$ACO_2H$ (III)

dans laquelle A conserve la même signification que dans la revendication 1, ou d'un dérivé fonctionnel de cet acide.

**18.** A titre de produits industriels nouveaux, les composés de formule (II) :

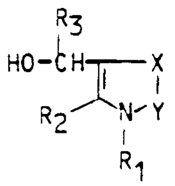

(II)

tels que définis à la revendication 17.

**19.** A titre de produit intermédiaire tel que défini à la revendication 17, la 5-hydroxyméthyl 3-(2-propynyl) 4-trifluorométhyl 2-(3H)-thiazolone.

**20.** Application des composés de formule (I) tels que définis à l'une quelconque des revendications 1 à 16 à la lutte contre les parasites des végétaux, les parasites des locaux et les parasites des animaux à sang chaud.

**21.** Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 16.

**22.** Les compositions insecticides renfermant comme principe actif au moins l'un des produits définis à l'une quelconque des revendications 1 à 16.

**23.** Les compositions acaricides renfermant comme principe actif au moins l'un des produits définis à l'une quelconque des revendications 1 à 16.

**24.** Les compositions nématicides renfermant comme principe actif au moins l'un des produits définis à l'une quelconque des revendications 1 à 16.

**25.** Les compositions acaricides destinées à la lutte contre les acariens parasites des animaux à sang chaud, notamment contre les tiques et les gales, caractérisées en ce qu'elles renferment comme principe actif, au moins l'un des produits définis à l'une quelconque des revendications 1 à 16.

**26.** Les compositions destinées à l'alimentation animale renfermant comme principe actif au moins l'un des produits définis à l'une quelconque des revendications 1 à 16.

**27.** Associations douées d'activité insecticide, acaricide ou nématicide, caractérisées en ce qu'elles contiennent comme matière active, d'une part un au moins des composés de formule générale (I) telle que définie à la revendication 1, et d'autre part, un au moins des ester pyréthrinoïdes choisis dans le groupe constitué par les esters d'alléthrolone, d'alcool 3,4,5,6-tétrahydro phtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcool alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzyliques des acides 2-parachlorophényl 2-isopropyl acétiques, par les esters

d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxybenzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

**28.** Les compositions telles que définies à l'une des revendications 21 à 27, caractérisées en ce qu'elles renferment en outre un synergiste des pyréthrinoïdes.

**Revendications pour les Etats contractants suivants : ES, GR**

**1.** Procédé de préparation des composés de formule (I) :

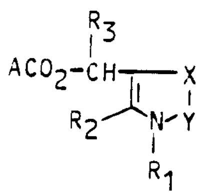

(I)

dans laquelle X représente un atome de soufre ou d'oxygène, Y représente un radical C = O, C = S ou $CH_2$, $R_1$ représente un atome d'hydrogène, un atome d'halogène, un radical alcoyle linéaire, ramifié ou cyclique, saturé ou insaturé, renfermant jusqu'à 8 atomes de carbone, éventuellement substitué par un ou plusieurs halogènes, ou un radical aryle renfermant jusqu'à 14 atomes de carbone, $R_2$ représente un atome d'hydrogène, un radical alcoyle linéaire, ramifié ou cyclique saturé ou insaturé, renfermant jusqu'à 8 atomes de carbone, éventuellement substitué par un ou plusieurs halogènes, un radical aryle renfermant jusqu'à 14 atomes de carbone, un radical $CF_3$, $NO_2$, C≡N, un atome d'halogène, un radical alcoxyle renfermant jusqu'à 8 atomes de carbone, $R_3$ représente un atome d'hydrogène, un radical alcoyle renfermant de 1 à 3 atomes de carbone, un radical C≡N ou un radical C≡CH et A représente le reste d'un acide pyréthrinoïde $ACO_2H$, sous toutes leurs formes stéréoisomères possibles, ainsi que des mélanges de ces stéréoisomères, caractérisé en ce que l'on soumet un alcool de formule (II) :

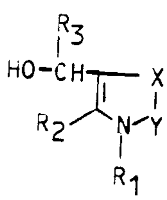

(II)

dans laquelle X, Y, $R_1$, $R_2$ et $R_3$ conservent la même signification que précédement, à l'action d'un acide de formule (III) :

$ACO_2H$ (III)

ou d'un dérivé fonctionnel de cet acide, dans lequel

- ou bien A représente un radical

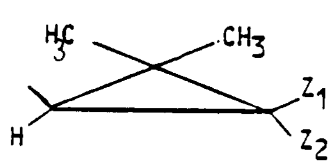

dans lequel :

- $Z_1$ et $Z_2$ représentent chacun un radical méthyle,

- ou $Z_1$ représente un atome d'hydrogène et
- soit $Z_2$ représente un radical

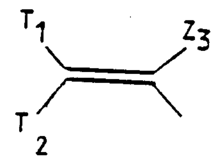

dans lequel $Z_3$ représente un atome d'hydrogène ou d'halogène et ou bien $T_1$ et $T_2$, identiques ou différents, représentent un atome d'hydrogène, un atome d'halogène, un radical alcoxyle ou alcoyle renfermant de 1 à 8 atomes de carbone, un radical $CF_3$, CN ou phényle éventuellement substitué par un halogène ou bien $T_1$ et $T_2$ forment ensemble un radical cycloalcoyle renfermant de 3 à 6 atomes de carbone ou un radical :

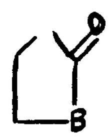

dans lequel B représente un atome d'oxygène ou de soufre ;

- soit $Z_2$ représente un radical :

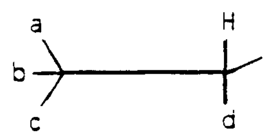

dans lequel a, b, c et d, identiques ou différents, représentent chacun un atome d'halogène,

- soit $Z_2$ représente un radical :

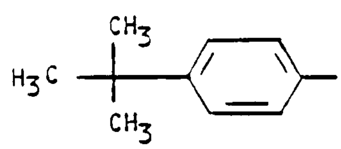

- soit $Z_2$ représente un radical :

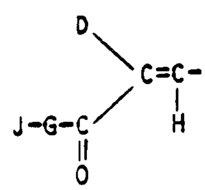

dans lequel D représente un atome d'hydrogène ou d'halogène, un radical alcoxyle renfermant de 1 à 8 atomes de carbone, G représente un atome d'oxygène ou de soufre et J représente ou bien un radical alcoyle linéaire, ramifié ou cyclique, saturé ou insaturé, renfermant de 1 à 8 atomes de carbone, éventuellement substitué par un ou plusieurs halogènes ou par des groupements fonctionnels identiques ou différents, choisis parmi les radicaux cyano, $OR_a$ dans lequel $R_a$ représente un atome d'hydrogène ou un radical alcoyle linéaire ou ramifié comportant de 1 à 8 atomes de carbone,

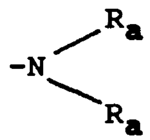

dans lequel $R_a$ est tel que défini ci-dessus, les deux radicaux $R_a$ pouvant être identiques ou différents ou 2-tétrahydropyrranyloxy, ou bien un groupement phényle ou bien un radical pyridyle, furyle, thiényle, oxazolyle ou thiazolyle,

- ou bien A représente un radical :

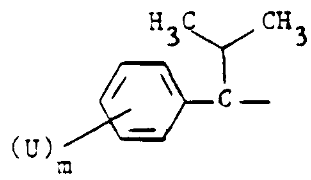

dans lequel U, en position quelconque sur le noyau benzénique, représente un atome d'halogène, un radical alcoyle renfermant de 1 à 8 atomes de carbone ou un radical alcoxy renfermant de 1 à 8 atomes de carbone, éventuellement substitués par 1 ou plusieurs atomes d'halogènes, m représente le nombre 0, 1 ou 2 et quand m est 2, les substituants peuvent être identiques ou différents,

- ou bien A représente un radical :

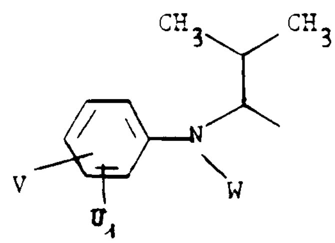

dans lequel $U_1$ et V identiques ou différents, représentent un atome d'hydrogène, un atome d'halogène ou un radical trifluorométhyle, W représente un atome d'hydrogène ou un radical méthyle.

**2.** Procédé selon la revendication 1, caractérisé en ce que l'on utilise au départ un alcool de formule (II) dans laquelle Y représente un radical C = O.

**3.** Procédé selon la revendication 1 ou 2, caractérisé en ce que l'on utilise au départ un alcool de formule (II) dans laquelle $R_3$ représente un atome d'hydrogène.

**4.** Procédé selon la revendication 1, 2 ou 3, caractérisé en ce que l'on utilise au départ un alcool de formule (II) dans laquelle $R_1$ représente un radical alcoyle insaturé renfermant jusqu'à 4 atomes de carbone.

**5.** Procédé selon la revendication 4, caractérisé en ce que $R_1$ est le radical 2-propynyle.

**6.** Procédé selon la revendication 4, caractérisé en ce que $R_1$ est le radical 2-propényle.

**7.** Procédé selon l'une quelconque des revendications 1 à 6, caractérisé en ce que l'on utilise au départ un alcool de formule (II) dans laquelle $R_2$ est le radical $CF_3$.

**8.** Procédé selon la revendication 1, caractérisé en ce que l'on utilise au départ un acide de formule (III) dans laquelle A représente le radical :

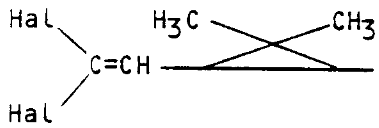

sous toutes les formes stéréoisomères possibles, ou sous forme de mélanges de ces stéréoisomères, Hal représentant un atome de fluor, de brome, de chlore ou d'iode.

**9.** Procédé selon la revendication 8, caractérisé en ce que Hal représente un atome de brome.

**10.** Procédé selon la revendication 1, caractérisé en ce que l'on utilise au départ un acide de formule (III) dans laquelle A représente le radical :

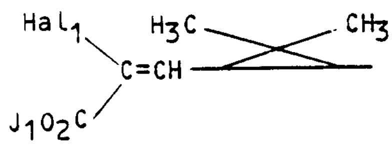

sous toutes les formes stéréoisomères possibles, ou sous forme de mélanges de ces stéréoisomères, dans lequel $Hal_1$ représente un atome d'halogène et $J_1$ représente un radical alcoyle saturé linéaire, ramifié ou cyclique, renfermant de 1 à 8 atomes de carbone, éventuellement substitué par un ou plusieurs halogènes, la double liaison ayant la géométrie E.

**11.** Procédé selon la revendication 10, caractérisé en ce que $Hal_1$ représente un atome de fluor.

**12.** Procédé selon la revendication 1, caractérisé en ce que l'on utilise au départ un acide de formule (III) dans laquelle A représente le radical :

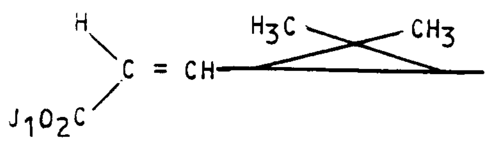

sous toutes les formes stéréoisomères possibles, ou sous forme de mélanges de ces stéréoisomères, dans lequel $J_1$ est défini comme la revendication 10, la double liaison ayant la géométrie Z.

**13.** Procédé selon la revendication 12, caractérisé en ce que $J_1$ représente un radical terbutyle.

**14.** Procédé selon la revendication 12, caractérisé en ce que $J_1$ représente un radical :

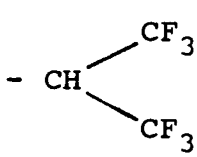

**15.** Procédé selon la revendication 1, caractérisé en ce que l'on utilise au départ un acide de formule (III) dans laquelle A représente le radical :

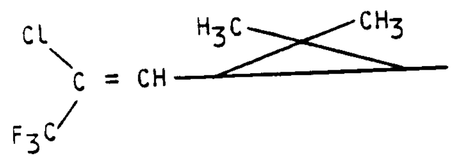

sous toutes ses formes stéréoisomères possibles, ou sous forme de mélanges de ces stéréoisomères.

## Claims

## Claims for the following Contracting States: CH, DE, FR, GB, IT, LI, NL

**1.** The subject of the invention is the compounds of formula (I):

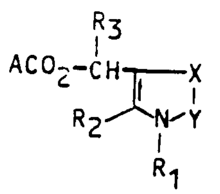

(I)

in which X represents a sulphur or oxygen atom, Y represents a C=O, C=S or $CH_2$ radical, $R_1$ represents a hydrogen atom, a halogen atom, a saturated or unsaturated, linear, branched or cyclic alkyl radical containing up to 8 carbon atoms, optionally substituted by one or more halogens, or an aryl radical containing up to 14 carbon atoms, $R_2$ represents a hydrogen atom, a saturated or unsaturated, linear, branched or cyclic alkyl radical containing up to 8 carbon atoms, optionally substituted by one or more halogens, an aryl radical containing up to 14 carbon atoms, a $CF_3$, $NO_2$, C≡N radical, a halogen atom, an alkoxyl radical containing up to 8 carbon atoms, $R_3$ represents a hydrogen atom, an alkyl radical containing 1 to 3 carbon atoms, a C≡N radical or a C≡CH radical and A represents the remainder of an $ACO_2H$ pyrethrinoid acid in which

- either A represents a radical

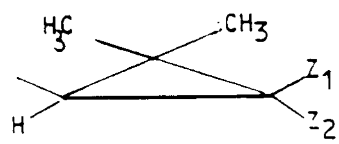

in which:

- $Z_1$ and $Z_2$ each represent a methyl radical,
- or $Z_1$ represents a hydrogen atom and
- either $Z_2$ represents a radical

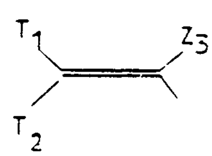

in which $Z_3$ represents a hydrogen or halogen atom and either $T_1$ and $T_2$, identical or different, represent a hydrogen atom, a halogen atom, an alkoxyl or alkyl radical containing 1 to 8 carbon atoms, a $CF_3$, CN or phenyl radical optionally substituted by a halogen or $T_1$ and $T_2$ together form a cycloalkyl radical containing 3 to 6 carbon atoms or a radical:

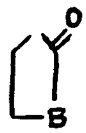

in which B represents an oxygen or sulphur atom;

- or $Z_2$ represents a radical:

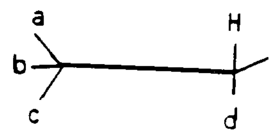

in which a, b, c and d, identical or different, each represent a halogen atom,

- or $Z_2$ represents a radical:

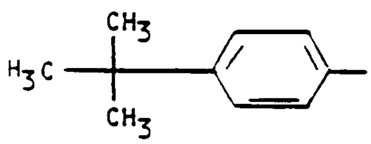

- or $Z_2$ represents a radical:

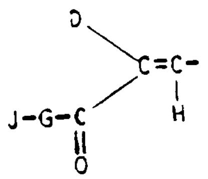

in which D represents a hydrogen or halogen atom, an alkoxyl radical containing 1 to 8 carbon atoms, G represents an oxygen or sulphur atom and J represents either a saturated or unsaturated, linear, branched or cyclic alkyl radical containing 1 to 8 carbon atoms, optionally substituted by one or more halogens or by identical or different functional groups, chosen from the following radicals: cyano, $OR_a$ in which $R_a$ represents a hydrogen atom or a linear or branched alkyl radical containing 1 to 8 carbon atoms,

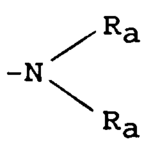

in which $R_a$ is as defined above, the two $R_a$ radicals being able to be identical or different, or 2-tetrahydropyranyloxy, or a phenyl group or a pyridyl, furyl, thienyl, oxazolyl or thiazolyl radical,

- or A represents a radical:

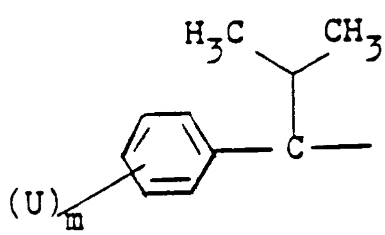

in which U, in whatever position on the benzene nucleus, represents a halogen atom, an alkyl radical containing 1 to 8 carbon atoms or an alkoxy radical containing 1 to 8 carbon atoms, optionally substituted by 1 or more halogen atoms, m represents the number 0, 1 or 2 and when m is 2, the substituents can be identical or different,

- or A represents a radical:

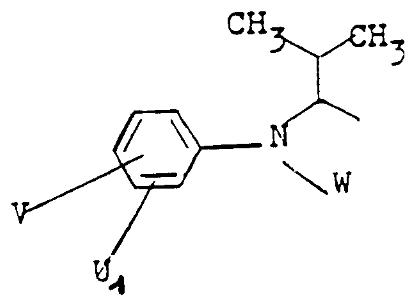

in which $U_1$ and V, identical or different, represent a hydrogen atom, a halogen atom or a trifluoromethyl radical, W represents a hydrogen atom or a methyl radical, in all their possible stereoisomer forms, as well as the mixtures of these stereoisomers.

**2.** The compounds of formula (I) as defined in claim 1 in which Y represents a C = O radical.

**3.** The compounds of formula (I) as defined in claim 1 or 2 in which $R_3$ represents a hydrogen atom.

**4.** The compounds of formula (I) as defined in claim 1, 2 or 3 in which $R_1$ represents an unsaturated alkyl radical containing up to 4 carbon atoms.

**5.** The compounds of formula (I) as defined in claim 4 in which $R_1$ is the 2-propynyl radical.

**6.** The compounds of formula (I) as defined in claim 4 in which $R_1$ is the 2-propenyl radical.

**7.** The compounds of formula (I) as defined in any one of claims 1 to 6 in which $R_2$ is the $CF_3$ radical.

**8.** The compounds of formula (I) as defined in any one of claims 1 to 7 in which A represents the radical:

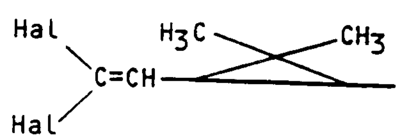

in all the possible stereoisomer forms, or in the form of mixtures of these stereoisomers, Hal representing a fluorine, bromine, chlorine or iodine atom.

**9.** The compounds of formula (I) as defined in claim 8, in which Hal represents a bromine atom.

**10.** The compounds of formula (I) as defined in any one of claims 1 to 7, in which A represents a radical:

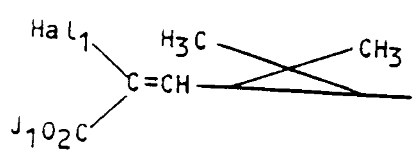

in all the possible stereoisomer forms, or in the form of mixtures of these stereoisomers, in which $Hal_1$

represents a halogen atom and $J_1$ represents a saturated linear, branched or cyclic alkyl radical containing 1 to 8 carbon atoms, optionally substituted by one or more halogens, the double bond having E geometry.

**11.** The compounds of formula (I) as defined in claim 10, in which $Hal_1$ represents a fluorine atom.

**12.** The compounds of formula (I) as defined in any one of claims 1 to 7 in which A represents the radical:

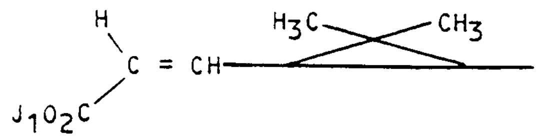

in all the possible stereoisomer forms, or in the form of mixtures of these stereoisomers, in which $J_1$ is defined as in claim 10, the double bond having Z geometry.

**13.** The compounds of formula (I) as defined in claim 12, in which $J_1$ represents a terbutyl radical.

**14.** The compounds of formula (I) as defined in claim 12 in which $J_1$ represents a radical:

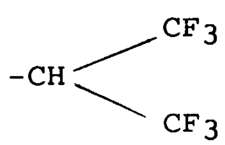

**15.** The compounds of formula (I) as defined in any one of claims 1 to 7 in which A represents a radical:

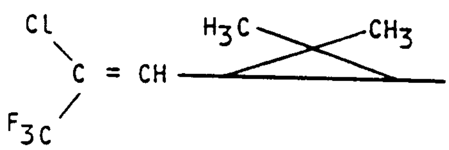

in all the possible stereoisomer forms, or in the form of mixtures of these stereoisomers.

**16.** The compounds of formula (I) as defined in claim 1, of which the names follow:

- (2-oxo-3-(2-propynyl)-4-trifluoromethyl-2,3-dihydrothiazol-5-yl) methyl [1R-[1alpha, 3alpha, (Z)]]-2,2-dimethyl-3-[2-chloro-3,3,3-trifluoropropenyl]-cyclopropane carboxylate;
- (2-oxo-3-(2-propynyl)-4-trifluoromethyl-2,3-dihydrothiazol-5-yl) methyl [1R-[1alpha, 3alpha, (Z)]]-2,2-dimethyl-3-[3-oxo-3-(1,1,1,3,3,3-hexafluoro-2-propyloxy]-1-propenyl]-cyclopropane carboxylate;
- (2-oxo-3-(2-propynyl)-4-trifluoromethyl-2,3-dihydrooxazol-5-yl) methyl [1R-[1alpha, 3alpha, (E)]]-2,2-dimethyl-3-[3-oxo-3-(1,1-dimethylethoxy)-2-fluoropropenyl]-cyclopropane carboxylate;
- (2-oxo-3-(2-propynyl)-2,3-dihydrothiazol-5-yl) methyl [1R-[1alpha, 3alpha, (E)]]-2,2-dimethyl-3-[2-fluoro-3-oxo-3-(2,2-dimethylethoxy)-1-propenyl]-cyclopropane carboxylate;
- and (2-oxo-3-(2-propynyl)-4-methyl-2,3-dihydrothiazol-5-yl) methyl [1R-[1alpha, 3alpha, (E)]-2,2-dimethyl-3-[2-fluoro-3-oxo-3-ethoxypropenyl]-cyclopropane carboxylate.

**17.** Preparation process for compounds of formula (I) as defined in any one of claims 1 to 16, characterized in that an alcohol of formula (II):

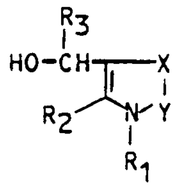

(II)

in which X, Y, $R_1$, $R_2$ and $R_3$ keep the same meaning as in claim 1, is subjected to the action of an acid of formula (III):

$ACO_2H$ (III)

in which A keeps the same meaning as in claim 1, or a functional derivative of this acid.

**18.** As new industrial products, the compounds of formula (II):

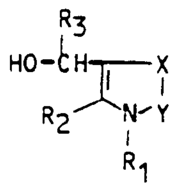

(II)

as defined in claim 17.

**19.** As an intermediate product as defined in claim 17, 5-hydroxymethyl-3-(2-propynyl)-4-trifluoromethyl-2-(3H)-thiazolone.

**20.** Use of compounds of formula (I) as defined in any one of claims 1 to 16 for combating parasites of vegetation, parasites of premises and parasites of warm-blooded animals.

**21.** The compositions intended for combating parasites of vegetation, parasites of premises and parasites of warm-blooded animals, characterized in that they contain as active ingredient at least one of the products defined in any one of claims 1 to 16.

**22.** The insecticide compositions containing as active ingredient at least one of the products defined in any one of claims 1 to 16.

**23.** The acaricide compositions containing as active ingredient at least one of the products defined in any one of claims 1 to 16.

**24.** The nematicide compositions containing as active ingredient at least one of the products defined in any one of claims 1 to 16.

**25.** The acaricide compositions intended for combating parasitic acaridae of warm-blooded animals, in particular ticks and mites, characterized in that they contain as active ingredient at least one of the products defined in any one of claims 1 to 16.

**26.** The compositions intended for animal fodder containing as active ingredient at least one of the products defined in any one of claims 1 to 16.

**27.** Combinations endowed with insecticide, acaricide or nematicide activity, characterized in that they contain as active ingredient, on the one hand at least one of the compounds of general formula (I) as defined in claim 1, and on the other hand, at least one of the pyrethrinoid esters chosen from the group constituted by the esters of allethrolone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohols with

chrysanthemic acids, by the esters of 5-benzyl-3-furyl methyl alcohol with 2,2-dimethyl-3-(2-oxo-3-tetrahydrothiophenylidene methyl)-cyclopropane-1-carboxylic acids, by the esters of 3-phenoxybenzyl alcohol and of alpha-cyano-3-phenoxybenzyl alcohol with 2,2-dimethyl-3-(2,2-dichlorovinyl)-cyclopropane-1-carboxylic acids, by the esters of alpha-cyano-3-phenoxy-benzyl alcohols with 2,2-dimethyl-3-(2,2-dibromovinyl)-cyclopropane-1-carboxylic acids, by the esters of 3-phenoxybenzyl alcohol with 2-parachlorophenyl-2-isopropyl acetic acids, by the esters of allethrolone, of 3,4,5,6-tetrahydrophthalimido methyl alcohol, of 5-benzyl-3-furyl methyl alcohol, of 3-phenoxybenzyl alcohol, and of alpha-cyano-3-phenoxybenzyl alcohols with 2,2-dimethyl-3-(1,2,2,2-tetrahaloethyl)-cyclopropane-1-carboxylic acids, in which "halo" represents a fluorine, chlorine or bromine atom, it being understood that the acid and alcohol copulas of the above pyrethrinoid esters can exist in all their possible stereoisomer forms.

**28.** The compositions as defined in one of claims 21 to 27, characterized in that they contain in addition a pyrethrinoid synergist.

**Claims for the following Contracting States: ES, GR**

**1.** Preparation process for compounds of formula (I):

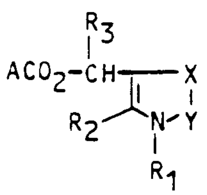

(I)

in which X represents a sulphur or oxygen atom, Y represents a C=O, C=S or $CH_2$ radical, $R_1$ represents a hydrogen atom, a halogen atom, a saturated or unsaturated, linear, branched or cyclic alkyl radical containing up to 8 carbon atoms, optionally substituted by one or more halogens, or an aryl radical containing up to 14 carbon atoms, $R_2$ represents a hydrogen atom, a saturated or unsaturated, linear, branched or cyclic alkyl radical containing up to 8 carbon atoms, optionally substituted by one or more halogens , an aryl radical containing up to 14 carbon atoms, a $CF_3$, $NO_2$, C≡N radical, a halogen atom, an alkoxyl radical containing up to 8 carbon atoms, $R_3$ represents a hydrogen atom, an alkyl radical containing 1 to 3 carbon atoms, a C≡N radical or a C≡CH radical and A represents the remainder of an $ACO_2H$ pyrethrinoid acid, in all their possible stereoisomer forms, as well as mixtures of these stereoisomers, characterized in that an alcohol of formula (II):

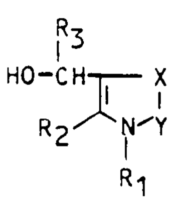

(II)

in which X, Y, $R_1$, $R_2$ and $R_3$ keep the same meaning as previously, is subjected to the action of an acid of formula (III):

$ACO_2H$ (III)

or a functional derivative of this acid, in which:

- either A represents a radical

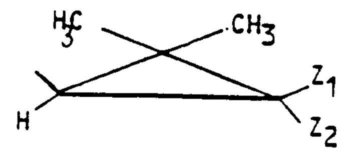

in which:

- $Z_1$ and $Z_2$ each represent a methyl radical,
- or $Z_1$ represents a hydrogen atom and
- either $Z_2$ represents a radical

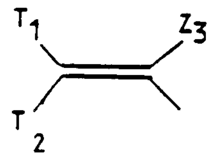

in which $Z_3$ represents a hydrogen or halogen atom and either $T_1$ and $T_2$, identical or different, represent a hydrogen atom, a halogen atom, an alkoxyl or alkyl radical containing 1 to 8 carbon atoms, a $CF_3$, CN or phenyl radical optionally substituted by a halogen or $T_1$ and $T_2$ together form a cycloalkyl radical containing 3 to 6 carbon atoms or a radical:

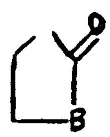

in which B represents an oxygen or sulphur atom;

- or $Z_2$ represents a radical:

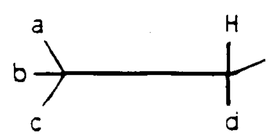

in which a, b, c and d, identical or different, each represent a halogen atom,

- or $Z_2$ represents a radical:

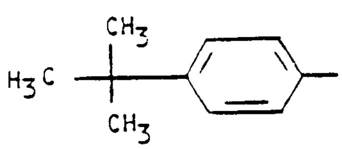

- or $Z_2$ represents a radical:

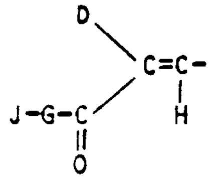

in which D represents a hydrogen or halogen atom, an alkoxyl radical containing 1 to 8 carbon atoms, G represents an oxygen or sulphur atom and J represents either a saturated or unsaturated, linear, branched or cyclic alkyl radical containing 1 to 8 carbon atoms, optionally substituted by one or more halogens or by identical or different functional groups, chosen from the following radicals: cyano, $OR_a$ in which $R_a$ represents a hydrogen atom or a linear or branched alkyl radical containing 1 to 8 carbon atoms,

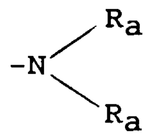

in which $R_a$ is as defined above, the two $R_a$ radicals being able to be identical or different, or 2-tetrahydropyranyloxy, or a phenyl group or a pyridyl, furyl, thienyl, oxazolyl or thiazolyl radical,

- or A represents a radical:

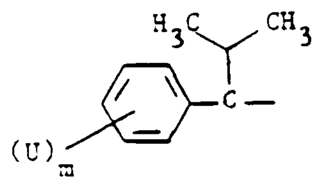

in which U, in whatever position on the benzene nucleus, represents a halogen atom, an alkyl radical containing 1 to 8 carbon atoms or an alkoxy radical containing 1 to 8 carbon atoms, optionally substituted by 1 or more halogen atoms, m represents the number 0, 1 or 2 and when m is 2, the substituents can be identical or different,

- or A represents a radical:

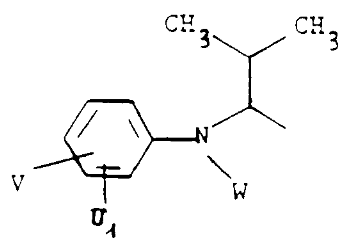

in which $U_1$ and V, identical or different, represent a hydrogen atom, a halogen atom or a trifluoromethyl radical, W represents a hydrogen atom or a methyl radical.

**2.** Process according to claim 1, characterized in that an alcohol of formula (II) is used at the start in which Y represents a C = O radical.

**3.** Process according to claim 1 or 2, characterized in that an alcohol of formula (II) is used at the start in which $R_3$ represents a hydrogen atom.

**4.** Process according to claim 1, 2 or 3, characterized in that an alcohol of formula (II) is used at the start in which $R_1$ represents an unsaturated alkyl radical containing up to 4 carbon atoms.

**5.** Process according to claim 4, characterized in that $R_1$ is the 2-propynyl radical.

**6.** Process according to claim 4, characterized in that $R_1$ is the 2-propenyl radical.

**7.** Process according to any one of claims 1 to 6, characterized in that an alcohol of formula (II) is used at the start in which $R_2$ is the $CF_3$ radical.

**8.** Process according to claim 1, characterized in that an acid of formula (III) is used at the start in which A represent the radical:

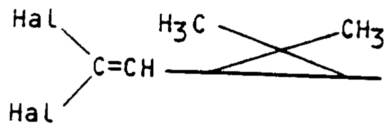

in all the possible stereoisomer forms, or in the form of mixtures of stereoisomers, Hal representing a fluorine, bromine, chlorine or iodine atom.

**9.** Process according to claim 8, characterized in that Hal represents a bromine atom.

**10.** Process according to claim 1, characterized in that an acid of formula (III) is used at the start in which A represent the radical:

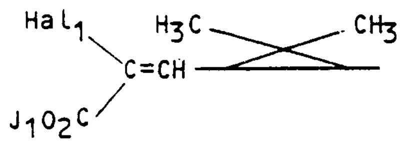

in all the possible stereoisomer forms, or in the form of mixtures of these stereoisomers, in which $Hal_1$ represents a halogen atom and $J_1$ represents a saturated, linear, branched or cyclic alkyl radical containing 1 to 8 carbon atoms, optionally substituted by one or more halogens, the double bond having E geometry.

**11.** Process according to claim 10, characterized in that $Hal_1$ represents a fluorine atom.

**12.** Process according to claim 1, characterized in that an acid of formula (III) is used at the start in which A represents the radical:

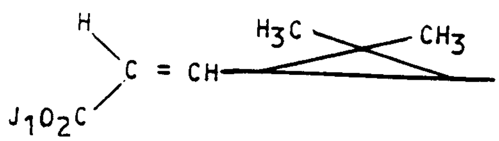

in all the possible stereoisomer forms, or in the form of mixtures of these stereoisomers, in which $J_1$ is defined as in claim 10, the double bond having Z geometry.

**13.** Process according to claim 12, characterized in that $J_1$ represents a terbutyl radical.

**14.** Process according to claim 12, characterized in that $J_1$ represents a radical:

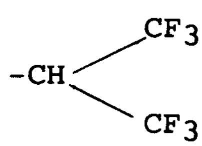

**15.** Process according to claim 1, characterized in that an acid of formula (III) is used at the start in which A represents the radical:

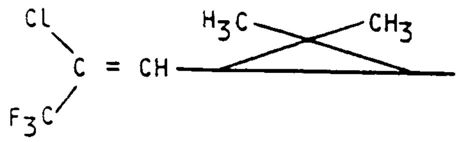

in all the possible stereoisomer forms, or in the form of mixtures of these stereoisomers.

**Patentansprüche**

**Patentansprüche für folgende Vertragsstaaten : CH, DE, FR, GB, IT, LI, NL**

**1.** Die Erfindung betrifft die Verbindungen der Formel (I)

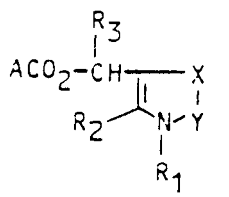

(I)

worin X für ein Schwefel- oder Sauerstoffatom steht, Y einen Rest C = O, C = S oder $CH_2$ bedeutet, $R_1$ ein Wasserstoffatom, ein Halogenatom, einen gesättigten oder ungesättigten, linearen, verzweigten oder cyclischen Alkylrest mit bis zu 8 Kohlenstoffatomen, der gegebenenfalls durch ein oder mehrere Halogenatome substituiert ist, oder einen Arylrest mit bis zu 14 Kohlenstoffatomen bedeutet, $R_2$ ein Wasserstoffatom, einen gesättigten oder ungesättigten, linearen, verzweigten oder cyclischen Alkylrest mit bis zu 8 Kohlenstoffatomen, der gegebenenfalls durch ein oder mehrere Halogenatome substituiert ist, einen Arylrest mit bis zu 14 Kohlenstoffatomen, einen Rest $CF_3$, $NO_2$, C≡N, ein Halogenatom, einen Alkoxyrest mit bis zu 8 Kohlenstoffatomen wiedergibt, $R_3$ für ein Wasserstoffatom, einen Alkylrest mit 1 bis 3 Kohlenstoffatomen, einen Rest C≡N oder einen Rest C≡CH steht und A den Rest einer Pyrethrinoidsäure $ACO_2H$ wiedergibt, worin

- entweder A einen Rest

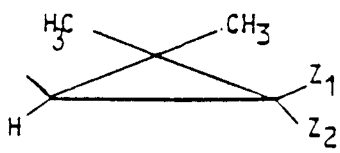

bedeutet, worin

- $Z_1$ und $Z_2$ jeweils einen Methylrest wiedergeben
- oder $Z_1$ für ein Wasserstoffatom steht und
- entweder $Z_2$ einen Rest

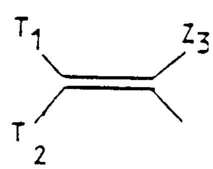

darstellt, worin $Z_3$ ein Wasserstoff- oder Halogenatom bedeutet und entweder $T_1$ und $T_2$, die gleich oder voneinander verschieden sind, ein Wasserstoffatom, ein Halogenatom, einen Alkoxyrest oder Alkylrest mit 1 bis 8 Kohlenstoffatomen, einen Rest $CF_3$, CN oder einen Phenylrest, der gegebenenfalls durch ein Halogenatom substituiert ist, bedeuten oder $T_1$ und $T_2$ gemeinsam einen Cycloalkylrest mit 3 bis 6 Kohlenstoffatomen oder einen Rest

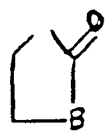

bilden, worin B für ein Sauerstoff- oder Schwefelatom steht;

- oder $Z_2$ einen Rest

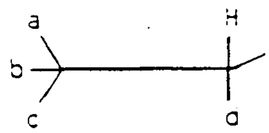

darstellt, worin a, b, c und d, die gleich oder voneinander verschieden sind, jeweils ein Halogenatom darstellen,

- oder $Z_2$ einen Rest

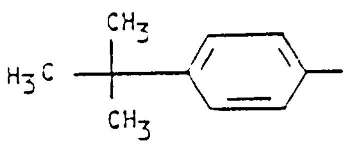

bedeutet

- oder $Z_2$ einen Rest

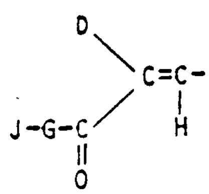

bedeutet, worin D für ein Wasserstoff- oder Halogenatom, einen Alkoxyrest mit 1 bis 8 Kohlenstoffatomen steht, G ein Sauerstoff- oder Schwefelatom wiedergibt und J entweder einen gesättigten oder ungesättigten, linearen, verzweigten oder cyclischen Alkylrest mit 1 bis 3 Kohlenstoffatomen, der gegebenenfalls durch ein oder mehrere Halogenatome oder durch gleiche oder voneinander verschiedene funktionelle Gruppen, ausgewählt unter den Cyanoresten, den Resten $OR_a$, worin $R_a$ für ein Wasserstoffatom oder einen linearen oder verzweigten Alkylrest mit 1 bis 8 Kohlenstoffatomen steht, den Resten

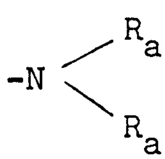 ,

worin $R_a$ wie vorstehend definiert ist, wobei beide Reste $R_a$ gleich oder voneinander verschieden sein können, oder der 2-Tetrahydropyranyloxygruppe,substituiert ist, oder eine Phenylgruppe oder einen Pyridyl-, Furyl-, Thienyl-, Oxazolyl- oder Thiazolylrest bedeutet,

- oder A einen Rest

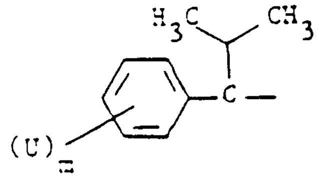

bedeutet, worin U in beliebiger Stellung an dem Benzolring für ein Halogenatom, einen Alkylrest mit 1 bis 8 Kohlenstoffatomen oder einen Alkoxyrest mit 1 bis 8 Kohlenstoffatomen, der gegebenenfalls durch ein oder mehrere Halogenatome substituiert ist, steht, m die Zahl 0, 1 oder 2 bedeutet, und wenn m für 2 steht, die Substituenten gleich oder voneinander verschieden sein können,

- oder A einen Rest

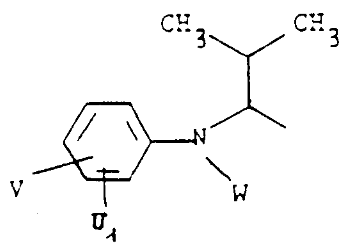

bedeutet, worin $U_1$ und V, die gleich oder voneinander verschieden sind, ein Wasserstoffatom, ein Halogenatom oder einen Trifluormethylrest wiedergeben, W ein Wasserstoffatom oder einen Methylrest bedeutet, in sämtlichen ihrer möglichen stereoisomeren Formen sowie die Gemische dieser Stereoisomeren.

**2.** Verbindungen der Formel (I), wie in Anspruch 1 definiert, worin Y für einen Rest C = O steht.

**3.** Verbindungen der Formel (I), wie in Anspruch 1 oder 2 definiert, worin $R_3$ ein Wasserstoffatom bedeutet.

**4.** Verbindungen der Formel (I), wie in Anspruch 1, 2 oder 3 definiert, worin $R_1$ einen ungesättigten Alkylrest mit bis zu 4 Kohlenstoffatomen bedeutet.

**5.** Verbindungen der Formel (I), wie in Anspruch 4 definiert, worin $R_1$ der 2-Propinylrest ist.

**6.** Verbindungen der Formel (I), wie in Anspruch 4 definiert, worin $R_1$ der 2-Propenylrest ist.

**7.** Verbindungen der Formel (I), wie in einem der Ansprüche 1 bis 6 definiert, worin $R_2$ der Rest $CF_3$ ist.

**8.** Verbindungen der Formel (I), wie in einem der Ansprüche 1 bis 7 definiert, worin A den Rest

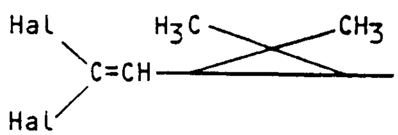

in sämtlichen der möglichen stereoisomeren Formen oder in Form von Gemischen dieser Stereoisomeren bedeutet, wobei Hal für ein Fluor-, Brom-, Chlor- oder Jodatom steht.

9. Verbindungen der Formel (I), wie in Anspruch 8 definiert, worin Hal für ein Bromatom steht.

10. Verbindungen der Formel (I), wie in einem der Ansprüche 1 bis 7 definiert, worin A für einen Rest

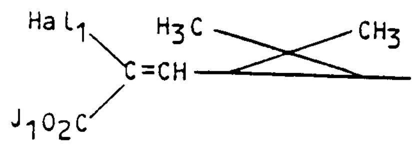

in sämtlichen der möglichen stereoisomeren Formen oder in Form von Gemischen dieser Stereoisomeren steht, worin $Hal_1$ ein Halogenatom bedeutet und $J_1$ einen linearen, verzweigten oder cyclischen, gesättigten Alkylrest mit 1 bis 8 Kohlenstoffatomen wiedergibt, der gegebenenfalls durch ein oder mehrere Halogenatome substituiert ist, wobei die Doppelbindung die E-Geometrie aufweist.

11. Verbindungen der Formel (I), wie in Anspruch 10 definiert, worin $Hal_1$ für ein Fluoratom steht.

12. Verbindungen der Formel (I), wie in einem der Ansprüche 1 bis 7 definiert, worin A für den Rest

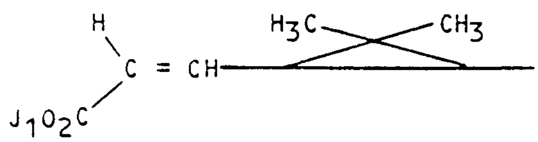

in sämtlichen der möglichen stereoisomeren Formen oder in Form von Gemischen dieser Stereoisomeren steht, worin $J_1$ wie in Anspruch 10 definiert ist, wobei die Doppelbindung die Z-Geometrie besitzt.

13. Verbindungen der Formel (I), wie in Anspruch 12 definiert, worin $J_1$ einen tert.-Butylrest bedeutet.

14. Verbindungen der Formel (I), wie in Anspruch 12 definiert, wobei $J_1$ einen Rest

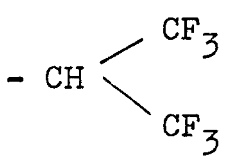

bedeutet.

15. Verbindungen der Formel (I), wie in einem der Ansprüche 1 bis 7 definiert, worin A einen Rest

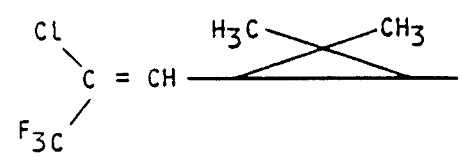

in sämtlichen seiner möglichen stereoisomeren Formen oder in Form von Gemischen dieser Stereoisomeren wiedergibt.

16. Die Verbindungen der Formel (I), wie in Anspruch 1 definiert, mit den folgenden Bezeichnungen:
(2-Oxo-3-(2-propinyl)-4-trifluormethyl-2,3-dihydrothiazol-5-yl)]-methyl-[1R-[1α,3α,(Z)]]-2,2-dimethyl-3-[2-chlor-3,3,3-trifluorpropenyl]-cyclopropancarboxylat,
(2-Oxo-3-(2-propinyl)-4-trifluormethyl-2,3-dihydrothiazol-5-yl)]-methyl-[1R-[1α,3α,(Z)]]-2,2-dimethyl-3-[3-oxo-3-(1,1,1,3,3,3-hexafluor-2-propyloxy]-1-propenyl]-cyclopropancarboxylat,

(2-Oxo-3-(2-Propinyl)-4-trifluormethyl-2,3-dihydrooxazol-5-yl)]-methyl-[1R-[1α,3α,(E)]]-2,2-dimethyl-3-[3-oxo-3-(1,1-dimethylethoxy)-2-fluorpropenyl]-cyclopropancarboxylat,

(2-Oxo-3-(2-Propinyl)-2,3-dihydrothiazol-5-yl)]-methyl-[1R-[1α,3α,(E)]]-2,2-dimethyl-3-[2-fluor-3-oxo-3-(2,2-dimethylethoxy)-1-propenyl]-cyclopropancarboxylat, und

(2-Oxo-3-(2-propinyl)-4-methyl-2,3-dihydrothiazol-5-yl)]-methyl-[1R-[1α,3α,(E)]]-2,2-dimethyl-3-[2-fluor-3-oxo-3-ethoxypropenyl)-cyclopropancarboxylat.

**17.** Verfahren zur Herstellung der Verbindungen der Formel (I), wie in einem der Ansprüche 1 bis 16 definiert, dadurch gekennzeichnet, daß man einen Alkohol der Formel (II)

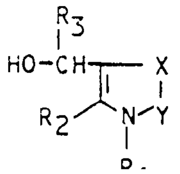

(II)

worin X, Y, $R_1$, $R_2$ und $R_3$ die in Anspruch 1 angegebene Bedeutung besitzen, der Einwirkung einer Säure der Formel (III)

$ACO_2H$ (III)

worin A die in Anspruch 1 angegebene Bedeutung besitzt, oder derjenigen eines funktionellen Derivats dieser Säure unterzieht.

**18.** Als neue industrielle Produkte die Verbindungen der Formel (II)

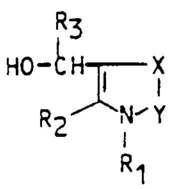

(II)

wie in Anspruch 17 definiert.

**19.** Als Zwischenprodukt, wie in Anspruch 17 definiert, das 5-Hydroxymethyl-3-(2-propinyl)-4-trifluormethyl-2-(3H)-thiazolon.

**20.** Verwendung der Verbindungen der Formel (I), wie in einem der Ansprüche 1 bis 16 definiert, bei der Bekämpfung von Schädlingen der Pflanzen, Schädlingen von Räumlichkeiten und Schädlingen warmblütiger Tiere.

**21.** Zusammensetzungen für die Bekämpfung von Schädlingen der Pflanzen, Schädlingen von Räumlichkeiten und Schädlingen warmblütiger Tiere, dadurch gekennzeichnet, daß sie als Wirkstoff zumindest eines der Produkte gemäß einem der Ansprüche 1 bis 16 enthalten.

**22.** Insektizide Zusammensetzungen, enthaltend als Wirkstoff zumindest eines der Produkte, wie in einem der Ansprüche 1 bis 16 definiert.

**23.** Akarizide Zusammensetzungen, enthaltend als Wirkstoff zumindest eines der Produkte, wie in einem der Ansprüche 1 bis 16 definiert.

**24.** Nematizide Zusammensetzungen, enthaltend als Wirkstoff zumindest eines der Produkte, wie in einem der Ansprüche 1 bis 16 definiert.

**25.** Akarizide Zusammensetzungen für die Bekämpfung von Milbenschädlingen warmblütiger Tiere, insbesondere von Zecken und Krätzmilben, dadurch gekennzeichnet, daß sie als Wirkstoff zumindest eines

der Produkte gemäß einem der Ansprüche 1 bis 16 enthalten.

**26.** Zusammensetzungen für die tierische Ernährung, enthaltend als Wirkstoff zumindest eines der Produkte gemäß einem der Ansprüche 1 bis 16.

**27.** Assoziationen mit insektizider,akarizider oder nematizider Aktivität, dadurch gekennzeichnet, daß sie als Wirkstoff einesteils zumindest eine der Verbindungen der allgemeinen Formel (I),wie in Anspruch 1 definiert,und anderenteils zumindest einen der Pyrethrinoidester,ausgewählt unter den Estern des Allethrolons, des 3,4,5,6-Tetrahydrophthalimidomethylalkohols,des 5-Benzyl-3-furylmethylalkohols, des 3-Phenoxybenzylalkohols und der α-Cyano-3-phenoxybenzylalkohole der Chrysanthemumsäuren, den Estern des 5-Benzyl-3-furylmethylalkohols der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren, den Estern des 3-Phenoxybenzylalkohols der 2-p-Chlorphenyl-2-isopropylessigsäuren, den Estern des Allethrolons, des 3,4,5,6-Teträhydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, enthalten, worin "halo" ein Fluor-, Chlor- oder Bromatom wiedergibt, mit der Maßgabe, daß die sauren Verknüpfungskomponenten und die Alkohole der vorstehenden Pyrethrinoidester in sämtlichen ihrer möglichen stereoisomeren Formen vorliegen können.

**28.** Zusammensetzungen, wie in einem der Ansprüche 21 bis 27 definiert, dadurch gekennzeichnet, daß sie außerdem einen Synergisten der Pyrethrinoide enthalten.

**Patentansprüche für folgende Vertragsstaaten : ES, GR**

**1.** Verfahren zur Herstellung der Verbindungen der Formel (I)

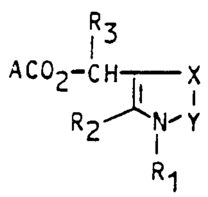

(I)

worin X für ein Schwefel- oder Sauerstoffatom steht, Y einen Rest C=O, C=S oder $CH_2$ bedeutet, $R_1$ ein Wasserstoffatom, ein Halogenatom, einen gesättigten oder ungesättigten, linearen, verzweigten oder cyclischen Alkylrest mit bis zu 8 Kohlenstoffatomen, der gegebenenfalls durch ein oder mehrere Halogenatome substituiert ist, oder einen Arylrest mit bis zu 14 Kohlenstoffatomen bedeutet, $R_2$ ein Wasserstoffatom, einen gesättigten oder ungesättigten, linearen, verzweigten oder cyclischen Alkylrest mit bis zu 8 Kohlenstoffatomen, der gegebenenfalls durch ein oder mehrere Halogenatome substituiert ist, einen Arylrest mit bis zu 14 Kohlenstoffatomen, einen Rest $CF_3$, $NO_2$, C≡N, ein Halogenatom, einen Alkoxyrest mit bis zu 8 Kohlenstoffatomen wiedergibt, $R_3$ für ein Wasserstoffatom, einen Alkylrest mit 1 bis 3 Kohlenstoffatomen, einen Rest C≡N oder einen Rest C≡CH steht und A den Rest einer Pyrethrinoidsäure $ACO_2H$ wiedergibt,
in sämtlichen ihrer möglichen stereoisomeren Formen sowie der Gemische dieser Stereoisomeren, dadurch **gekennzeichnet,** daß man einen Alkohol der Formel (II)

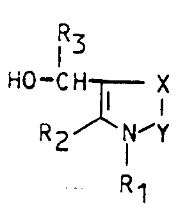

(II)

worin X, Y, $R_1$, $R_2$ und $R_3$ die vorstehend angegebene Bedeutung besitzen, der Einwirkung einer Säure

der Formel (III)

$ACO_2H$ (III)

oder eines funktionellen Derivats dieser Säure unterzieht, worin

- entweder A einen Rest

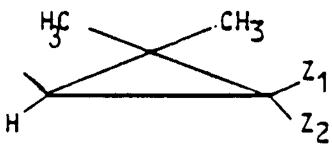

bedeutet, worin

- $Z_1$ und $Z_2$ jeweils einen Methylrest wiedergeben
- oder $Z_1$ für ein Wasserstoffatom steht und
- entweder $Z_2$ einen Rest

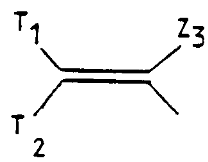

darstellt, worin $Z_3$ ein Wasserstoff- oder Halogenatom bedeutet und entweder $T_1$ und $T_2$, die gleich oder voneinander verschieden sind, ein Wasserstoffatom, ein Halogenatom, einen Alkoxyrest oder Alkylrest mit 1 bis 8 Kohlenstoffatomen, einen Rest $CF_3$, CN oder einen Phenylrest, der gegebenenfalls durch ein Halogenatom substituiert ist, bedeuten oder $T_1$ und $T_2$ gemeinsam einen Cycloalkylrest mit 3 bis 6 Kohlenstoffatomen oder einen Rest

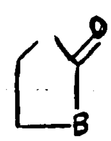

bilden, worin B für ein Sauerstoff- oder Schwefelatom steht;

- oder $Z_2$ einen Rest

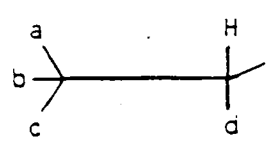

darstellt, worin a, b, c und d, die gleich oder voneinander verschieden sind, jeweils ein Halogenatom darstellen,

- oder $Z_2$ einen Rest

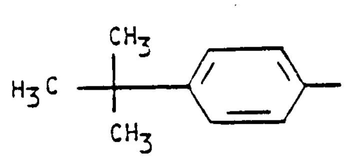

bedeutet
- oder $Z_2$ einen Rest

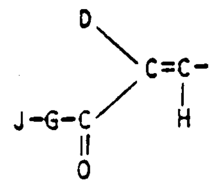

bedeutet, worin D für ein Wasserstoff- oder Halogenatom, einen Alkoxyrest mit 1 bis 8 Kohlenstoffatomen steht, G ein Sauerstoff- oder Schwefelatom wiedergibt und J entweder einen gesättigten oder ungesättigten, linearen, verzweigten oder cyclischen Alkylrest mit 1 bis 8 Kohlenstoffatomen, der gegebenenfalls durch ein oder mehrere Halogenatome oder durch gleiche oder voneinander verschiedene funktionelle Gruppen, ausgewählt unter den Cyanoresten, den Resten $OR_a$, worin $R_a$ für ein Wasserstoffatom oder einen linearen oder verzweigten Alkylrest mit 1 bis 8 Kohlenstoffatomen steht, den Resten

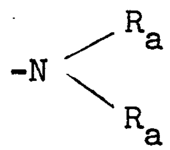 ,

worin $R_a$ wie vorstehend definiert ist, wobei beide Reste $R_a$ gleich oder voneinander verschieden sein können, oder der 2-Tetrahydropyranyloxygruppe,substituiert ist, oder eine Phenylgruppe oder einen Pyridyl-, Furyl-, Thienyl-, Oxazolyl- oder Thiazolylrest bedeutet,
- oder A einen Rest

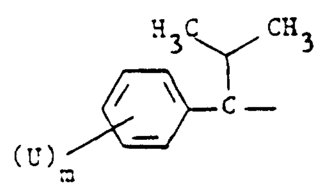

bedeutet, worin U in beliebiger Stellung an dem Benzolring für ein Halogenatom, einen Alkylrest mit 1 bis 8 Kohlenstoffatomen oder einen Alkoxyrest mit 1 bis 8 Kohlenstoffatomen, der gegebenenfalls durch ein oder mehrere Halogenatome substituiert ist, steht, m die Zahl 0, 1 oder 2 bedeutet, und wenn m für 2 steht, die Substituenten gleich oder voneinander verschieden sein können,
- oder A einen Rest

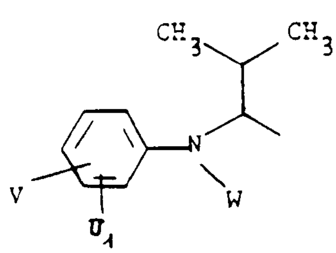

bedeutet, worin $U_1$ und V, die gleich oder voneinander verschieden sind, ein Wasserstoffatom, ein Halogenatom oder einen Trifluormethylrest wiedergeben, W ein Wasserstoffatom oder einen Methylrest bedeutet.

**2.** Verfahren gemäß Anspruch 1, dadurch gekennzeichnet, daß man von einem Alkohol der Formel (II) ausgeht, worin Y für einen Rest C = O steht.

**3.** Verfahren gemäß Anspruch 1 oder 2, dadurch gekennzeichnet, daß man von einem Alkohol der Formel (II) ausgeht, worin $R_3$ ein Wasserstoffatom bedeutet.

**4.** Verfahren gemäß Anspruch 1, 2 oder 3, dadurch gekennzeichnet, daß man von einem Alkohol der Formel (II) ausgeht, worin $R_1$ für einen ungesättigten Alkylrest mit bis zu 4 Kohlenstoffatomen steht.

**5.** Verfahren gemäß Anspruch 4, dadurch gekennzeichnet, daß $R_1$ der 2-Propinylrest ist.

**6.** Verfahren gemäß Anspruch 4, dadurch gekennzeichnet, daß $R_1$ der 2-Propenylrest ist.

**7.** Verfahren gemäß einem der Ansprüche 1 bis 6, dadurch gekennzeichnet, daß man von einem Alkohol der Formel (II) ausgeht, worin $R_2$ der Rest $CF_3$ ist.

**8.** Verfahren gemäß Anspruch 1, dadurch gekennzeichnet, daß man von einer Säure der Formel (III) ausgeht, worin A den Rest

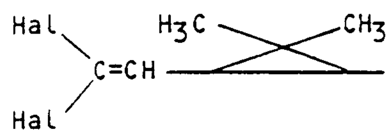

in sämtlichen der möglichen stereoisomeren Formen oder in Form von Gemischen dieser Stereoisomeren wiedergibt, wobei Hal ein Fluor-, Brom-, Chlor- oder Jodatom bedeutet.

**9.** Verfahren gemäß Anspruch 8, dadurch gekennzeichnet, daß Hal für ein Bromatom steht.

**10.** Verfahren gemäß Anspruch 1, dadurch gekennzeichnet, daß man von einer Säure der Formel (III) ausgeht, worin A für den Rest

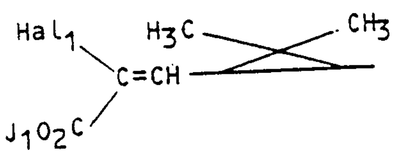

in sämtlichen der möglichen stereoisomeren Formen oder in Form von Gemischen dieser Stereoisomeren steht, worin $Hal_1$ ein Halogenatom bedeutet und $J_1$ für einen linearen, verzweigten oder cyclischen, gesättigten Alkylrest mit 1 bis 8 Kohlenstoffatomen steht, der gegebenenfalls durch ein oder mehrere Halogenatome substituiert ist, wobei die Doppelbindung die E-Geometrie aufweist.

**11.** Verfahren gemäß Anspruch 10, dadurch gekennzeichnet, daß $Hal_1$ für ein Fluoratom steht.

**12.** Verfahren gemäß Anspruch 1, dadurch gekennzeichnet, daß man von einer Säure der Formel (III) ausgeht, worin A den Rest

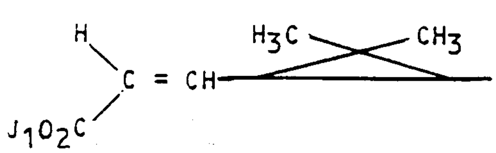

in sämtlichen der möglichen stereoisomeren Formen oder in Form von Gemischen dieser Stereoisomeren wiedergibt, worin $J_1$ wie in Anspruch 10 definiert ist, wobei die Doppelbindung die Z-Geometrie

aufweist.

**13.** Verfahren gemäß Anspruch 12, dadurch gekennzeichnet, daß $J_1$ einen tert.-Butylrest bedeutet.

**14.** Verfahren gemäß Anspruch 12, dadurch gekennzeichnet, daß $J_1$ einen Rest

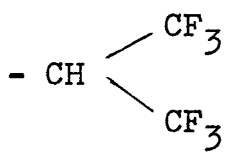

wiedergibt.

**15.** Verfahren gemäß Anspruch 1, dadurch gekennzeichnet, daß man von einer Säure der Formel (III) ausgeht, worin A für den Rest

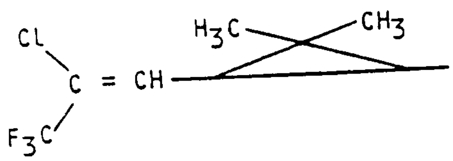

in sämtlichen seiner möglichen stereoisomeren Formen oder in Form von Gemischen dieser Stereoisomeren steht.